# EUROPEAN PATENT SPECIFICATION

(11) **EP 1 131 421 B1**
(45) Date of publication and mention of the grant of the patent: **19.07.2006**
(21) Application number: 99972677.1
(22) Date of filing: 18.11.1999
(51) Int. Cl.: C12N 15/10, C12N 15/13, C12N 15/55, C12N 5/10, C07K 16/00

(54) **METHODS FOR THE PREPARATION BY IN VIVO RECOMBINATION OF NUCLEIC ACID AND POLYPEPTIDE LIBRARIES AND USES THEREOF**
VERFAHREN ZUR HERSTELLUNG VON NUKLEINSÄURE- UND POLYPEPTIDBANKEN DURCH IN VIVO REKOMBINATION UND IHRE VERWENDUNGEN
METHODES DE PREPARATION DE BANQUES D'ACIDES NUCLEIQUES ET DE POLYPEPTIDES ET UTILISATIONS DE TELLES BANQUES

(30) Priority: 19.11.1998 IT MI982509
(43) Date of publication of application: 12.09.2001
(73) Proprietor: S.I.S.S.A. Scuola Internazionale Superiore di Studi Avanzati, 34014 Trieste (IT)
(72) Inventor: BRADBURY, Andrew, Raymon, Morton, Santa Fe, NM 87501 (US); SBLATTERO, Daniele, I-33085 Maniago (IT)
(74) Representative: Capasso, Olga
(86) International application number: PCT/EP1999/008856
(87) International publication number: WO 2000/031246

(56) References cited:
- WO-A-97/20078
- WO-A-98/28416
- US-A- 5 728 557
- GRIFFITHS A D ET AL: "ISOLATION OF HIGH AFFINITY HUMAN ANTIBODIES DIRECTLY FROM LARGE SYNTHETIC REPERTOIRES" EMBO JOURNAL,GB,OXFORD UNIVERSITY PRESS, SURREY, vol. 13, no. 14, 15 July 1994 (1994-07-15), pages 3245-3260, XP000455240 ISSN: 0261-4189 cited in the application
- DAVIES JULIAN ET AL: "An antibody VH domain with a lox-Cre site integrated into its coding region: Bacterial recombination within a single polypeptide chain." FEBS LETTERS 1995, vol. 377, no. 1, 1995, pages 92-96, XP000891732 ISSN: 0014-5793
- TSURUSHITA N ET AL: "Phage display vectors for in vivo recombination of immunoglobulin heavy and light chain genes to make large combinatorial libraries" GENE,NL,ELSEVIER BIOMEDICAL PRESS. AMSTERDAM, vol. 172, no. 1, 12 June 1996 (1996-06-12), pages 59-63, XP004042709 ISSN: 0378-1119
- GEOFFROY F ET AL: "A NEW PHAGE DISPLAY SYSTEM TO CONSTRUCT MULTICOMBINATORIAL LIBRARIES OF VERY LARGE ANTIBODY REPERTOIRES" GENE,NL,ELSEVIER BIOMEDICAL PRESS. AMSTERDAM, vol. 151, no. 1/02, 1 January 1994 (1994-01-01), pages 109-113, XP000579650 ISSN: 0378-1119 cited in the application
- BETT ET AL: "AN EFFICIENT AND FLEXIBLE SYSTEM FOR CONSTRUCTION OF ADENOVIRUS VECTORS WITH INSERTIONS OR DELETIONS IN EARLY REGIONS 1 AND 3" PROCEEDINGS OF THE NATIONAL ACADEMY OF SCIENCES OF USA,US,NATIONAL ACADEMY OF SCIENCE. WASHINGTON, vol. 91, no. 91, September 1994 (1994-09), pages 8802-8806-8806, XP002126553 ISSN: 0027-8424
- SHEETS MICHAEL D ET AL: "Efficient construction of a large nonimmune phage antibody library: The production of high-affinity human single-chain antibodies to protein antigens." PROCEEDINGS OF THE NATIONAL ACADEMY OF SCIENCES OF THE UNITED STATES OF AMERICA MAY 26, 1998, vol. 95, no. 11, 26 May 1998 (1998-05-26), pages 6157-6162, XP002134533 ISSN: 0027-8424
- GU H ET AL: "INDEPENDENT CONTROL OF IMMUNOGLOBULIN SWITCH RECOMBINATION AT INDIVIDUAL SWITCH REGIONS EVIDENCED THROUGH CRE-LOX-P-MEDIATED GENE TARGETING" CELL,US,CELL PRESS, CAMBRIDGE, NA, vol. 73, 18 June 1993 (1993-06-18), pages 1155-1164, XP000749187 ISSN: 0092-8674
- SBLATTERO D ET AL: "Exploiting recombination in single bacteria to make large phage antibody libraries." NATURE BIOTECHNOLOGY, (2000 JAN) 18 (1) 75-80. , XP000891515

## Description

### Field of the invention

The present invention refers to a procedure for the generation of genetic diversity through the preparation of gene libraries and to the use of said gene libraries for the preparation of new polypeptides and to the polypeptides obtained in this way.

### Background of the invention

The creation of diversity in populations of polypeptides has become an important tool in the derivation of polypeptides with useful characteristics. Such approaches typically involve methods to create diverse populations of molecules (*e.g.*, nucleic acids, polypeptides, *etc.)* coupled to methods to select or screen for population members that possess one or more desired characteristics.

A number of approaches have been used to create diverse populations of molecules. Such methods include random mutagenesis, recombinatorial mutagenesis, and directed mutagenesis. In random mutagenesis, mutations are typically inserted into a DNA sequence at random. This can be carried out using a number of different methods including chemical mutagenesis, mutator bacterial strains and error prone PCR. In recombinatorial mutagenesis recombination occurs between different DNA strands in such a way that mutations, differences or genes that are originally present on different DNA strands are brought together on the same strand. In directed mutagenesis, mutations are inserted into a DNA sequence at sites in which the encoded protein is known, or suspected, to participate in particular processes such as binding or enzymatic activities. This can be done by site directed mutagenesis or PCR.

Of these three forms of mutagenesis, recombinatorial mutagenesis and directed mutagenesis appear to be the most efficient. However, directed mutagenesis requires detailed structural knowledge of the target protein and/or nucleic acid.

Recombinatorial mutagenesis with the goal of making gene libraries has only been carried out *in vitro* using DNA shuffling (Stemmer (1994) *Proc Natl Acad Sci USA,* 91: 10747-10751.). By using DNA shuffling, cloning and transfection, a number of different proteins, including beta lactamase, galactosidase and antibodies have had their properties modified (in a directed manner).

By using closely related genes in DNA shuffling, rather than mutations accumulated during PCR, novel proteins have been produced which have properties not found in any of the proteins encoded by the genes used as substrates for shuffling. Examples include interferons and cephalosporinases (Crameri, *et al.* (1998) *Nature,* 391: 288-91.).

One particular application in which diversity is created by the combinatorial assortment of two different genes, rather than recombination within a single gene or set of genes is the creation of antibody libraries displayed on the surface of filamentous phage or phagemid (phage display) (Marks *et al.* (1991) *J. Mol. Biol.,* 222, 581-597.).

The binding domain of antibodies is encoded in the V regions, with each binding domain being made up of a single VH domain and a single V_{L} domain. It has proved possible to separate the V domains from the rest of the immunoglobulin and create novel functional binding polypeptides. Fv fragments are made up of V_{H} and V_{L} and are held together by non-covalent forces. However they are relatively unstable, and the two chains easily separate. Joining the two chains with a polypeptide linker creates the single chain Fv (scFv) fragment which is far more stable.

The Fab fragment consists of V_{H} and C_{H}1 coupled covalently via disulfide bonds to V_{L} and

C_{L}. This fragment is stable, but suffers from the disadvantage that it is relatively more difficult to make in bacteria and has a tendency to aggregate. Most libraries made on filamentous phage are in the scFv format, although a few have also been made in the Fab format.

Although antibodies against a large number of antigens have been isolated using phage antibody libraries *(see, e.g.*, (Marks *et al.* (1991) *J. Mol. Biol.,* 222, 581-597.; Griffiths, *et al.* (1994) *EMBO J.,* 13, 3245-3260.)), the procedure is still not yet widely used. This may be due to the difficulty of making large phage antibody libraries, which typically require that a library of at least 10⁹ independent clones is created from a good source of diverse VH and VL genes. In general, such libraries are made by carrying out a large number of ligations and transfections, and once made, become a limited resource as amplification cannot be carried out without a potential reduction in diversity. In general, the affinity of the antibodies isolated is proportional to the initial size of the library used for selection.

Site specific recombination has been proposed as an alternative method to the use of cloning to create large gene libraries, and for the preparation of antibodies with new specificities. To recombine V_{H} and V_{L} chains in the Fab (Griffiths et al. 1994) or in the scFv (Tsurushita *et al.* (1996) *Gene* 172: 59-63) formats, cre recombinase has been used while in another case lambda recombinase has been used to recombine Fab (Geoffroy *et al.,* (1994) *Gene* 151:109-113)*.*

While recombination between single antibodies to reconstitute functional binding regions was described in all of these reports, a large phage antibody library has only been made using cre recombinase to recombine Fabs (*i.e.* site specific recombination) described in WO93/19172 and (Griffiths, *et al.* (1994) *EMBO J.,* 13: 3245-3260.).

In both of these publications a diverse gene library was prepared using site-specific recombination between a first vector containing the V_{H} gene and a second vector containing the V_{L} gene, with the resultant recombined vector containing both V_{H} and V_{L} genes. These systems used two different vectors for recombination.

The introduction of the different expression vectors carrying the genes to be recombined within the cell, however, posed serious problems to the creation of large diverse libraries. Typically, the two vectors had to be different (*e.g.* on e vector a plasmid, the other vector a phage). This is because it had been reported that bacteria infected by a filamentous phage are resistant to further infection by other filamentous phage (Boeke *et al.* (1982) *Mol Gen Genet* 186: 185-192). One of the two vectors thus had to be subject to transfection rather than infection and this drastically lowered the efficiency of transformation of the cell.

In addition, it was believed that the two constructs required different origins of replication belonging to different incompatibility groups and also different antibiotic resistance genes. This is because it was believed by experts in the field that plasmids that utilize the same replication system (origin of replication) cannot co-exist stably in a cell are said to be incompatible (see pages 1.3-4 of (Sambrook *et al.* (1989) *Molecular cloning: a laboratory manual,* Cold Spring Harbor Laboratory Press)). For each plasmid and origin of replication, a different antibiotic resistance gene was also required. This limited the total number of different plasmids which could be maintained within a single cell. Consequently, the number of recombination substrates that could be present within a single cell was limited to the number of different origins of replication and antibiotic resistance genes that could be used. Thus, the size and diversity of a library that could be made using this approach was seriously limited.

The use of the "two-construct" (plasmid +phage) approach also provided other problems limiting its use. First, the two-construct recombination system is reversible, and as a result libraries produced using this system are "contaminated" with the starting phage (*e.g.*, containing dummy V_{H} chains) and plasmids (containing the V_{H}+C_{H}1 library or the dummy V_{H}+CH₁) at relatively high levels. The host cells were thus, overloaded with "useless" constructs and the system requires substantial resources (*e.g.* host cells) to maintain significant levels of "useful" (properly recombined) constructs.

A second problem is that the plasmids, even though they do not contain an Ff origin of replication, can nevertheless be incorporated into phage particles with variable efficiencies. As a result, the final library contains a mixture of many different genetic elements, so reducing the effective functional diversity.

A third problem was that recombination occurs between a single phage DNA and a single plasmid DNA molecule in a host cell and recombination between multiple different plasmids was not possible. As a result, each bacteria (host cell) produced a single novel recombined antibody. This limited the size of the library obtained to the number of bacteria used in the recombination step, which reaches a practical limit of 5x10¹² for 10 liters). To obtain larger libraries, more bacteria need to be used.

A fourth limitation was that the products of recombination in such a system are "final" products, and cannot be recombined again. As a result there is no facility to undergo further recombination between: already recombined V_{H} and V_{L} genes or recombined V_{H} genes and the library of V_{L} genes. Only recombined V_{L} genes could be recombined with the original V_{H} gene library.

### Summary of the invention

The methods and constructs of the present invention overcome these and other problems and allow the creation of large highly diverse libraries of nucleic acids and/or polypeptides. The methods exploit the discovery that recombination, particularly recombinase recombination can occur between two or more constructs of the same type (*e.g.* having the same origin of replication and/or the same regulatory or selectable markers). The methods thus involve introducing at least two members of an initial population of nucleic acid molecules into at least one cell (*e.g.* a bacterial, plant, yeast, insect, fungal, vertebrate, mammalian cell, *etc.).* The nucleic acid molecules preferably comprise a nucleic acid sequence that is identical for each molecule (member of the library) and that includes an origin of replication (and optionally other regulatory sequences, and/or vector backbone, *etc.) ;* and a nucleic acid sequence that varies between members of the population and comprises at least one substrate for recombination. Once in the cell the members of the library recombine such that at least one substrate for recombination recombines between at least two members of the population thereby producing a population comprising recombined nucleic acid members. The recombination can be performed by a recombination mechanism endogenous to the cell (*e.g.* general recombination or recombinase mediated recombination) or by a recombinase exogenous to the cell (*e.g.* externally provided recombinase or by transfection with a recombinase-expressing nucleic acid). Typically the members of the library have at least two recombinase recognition sites and recombination preferably occurs at site preselected for recombination. In preferred embodiments, the recombination is mediated by a recombinase selected from the group consisting of a member of the hin family of recombinases, a member of the lambda integrase family, an flp recombinase, a resolvase, a transposon, and a Cre recombinase and in most preferred embodiments, the recombinases include, but are not limited to of Cre, hin, gin, pin, cin, and flp. In a most preferred embodiment recombination is at a loxP site or a flp site.

In certain embodiments a substrate for recombination comprises (*e.g.* is flanked) by a pair of recombination recognition sites (*e.g.* a first and a second recombination recognition site) and these sites can be the same or different. In particularly preferred embodiments, the recombination results in the exchange, of at least one recombination substrate between at least two members of the nucleic acid population. One preferred pair of recombination recognition sites is loxP and a loxP mutant (*e.g*. loxP 511 or fas loxP).

The members of the nucleic acid library can be introduced into the cell by any convenient method including biolistics, transfection, or infection (*e.g.,* when contained within infectious particles): Preferred populations of nucleic acid molecules comprise at least 2 different members, preferably at least 10 different members, more preferably at least 50 different members, and most preferably at least 500 different members per cell *(i.e.* transfected and/or infected into a cell). Preferred nucleic acid libraries comprise at least 10⁵, preferably at least 10⁷, more preferably at least 10⁸, 10⁹, or 10¹⁰ different members, and most preferably at least, 10¹¹, 10¹², 10¹³, 10¹⁴, 10¹⁵ or more different members. Typically (although not so restricted) libraries before recombination will comprise 10⁶-10⁸ different members and after recombination will comprise 10⁹-10¹³ or more members. Preferred infectious particles used in this method include, but are not limited to phage (*e.g.* filamentous phage such as phage of the Ff family) and phagemid (*e.g.*, phagemid derived from members of the phage Ff family)..

The methods of this invention can additionally involve transfecting or infecting one or more cells with members of the population of recombined nucleic acid members such that the cells are infected at a multiplicity of infection (moi) of less than about 1; and packaging the members of the nucleic acid library in replicable genetic display packages such that a protein on the surface of the replicable display package is encoded by a nucleic acid packaged within the display package that is a nucleic acid sequence that varies between members of the nucleic acid library.

In particularly preferred embodiments, the variable nucleic acid sequence comprising the substrate for recombination in the members of the nucleic acid libraries comprises an expression cassette, preferably an expression cassette comprising a nucleic acid sequences encoding one or more polypeptides. In a most preferred embodiment, the nucleic acid encoding at least one of the polypeptides is flanked by pair of recombinase recognition sites (*e.g.*, where the members of the pair of recognition sites are the same or different as described above).

Preferred expression cassettes express the polypeptides on the surface of a phage, a phagemid, or a bacterium. In one embodiment, the variable sequence (*e.g.* the expression cassette) can include a nucleic acid sequence encoding a first polypeptide chain and nucleic acid sequence encoding a second polypeptide chain where the polypeptide chains are from a specific binding pair member *(e.g.* an antibody, a receptor, *etc.)* such that following recombination the variable sequence encodes binding proteins (or pairs of binding proteins) that are not encoded for in the initial population of nucleic acids._ In this instance preferred first and said second polypeptides include antibody polypeptides (*e.g.* antibody fragments/domains). Particularly preferred antibody fragments include, but are not limited to a V_{H} region, a V_{L} region, a V_{H} CDR1, a V_{H} CDR2, a V_{H} CDR3, a V_{L} CDR1, a V_{L} CDR2, a V_{L} CDR3, a V_{H} joined to a C_{H}1, a V_{L} joined to a C_{L}, and the like. Where the two polypeptides form an scFv, the first polypeptide is a V_{H} region and the second polypeptide is a V_{L} region. Typically at least one recombinase recognition site will be present in the nucleic acid encoding a linker joining the V_{H} and V_{L} regions. The V_{H} and V_{L} regions may be in the order V_{L} followed by V_{H} or *vice versa.* Such recombinase sites will often be members of a pair of recombinase recognition sites flanking the nucleic acid encoding a first polypeptide and the recombinase sites forming the pair may be the same or different as explained above. Both polypeptides of any combination of antibody fragments comprising an antibody can have one or more of the substituent fragments flanked by a pair of recombinase recognition sites. Particular preferred members of the nucleic acid library encode single chain antibodies (*e.g*., scFv). Other preferred constructs include, but are not limited to a Fab, a diabody, a V_{H} dimer, and a V_{L} dimer, and the like.

In another embodiment this invention provides nucleic acid libraries made by the methods of this invention.

This invention also provides a nucleic acid library comprising a population of nucleic acid molecules comprising two or more individual nucleic acids each of which consists of a nucleic acid sequence that is identical for each molecule and that includes an origin of replication and at least two recombinase recognition sites; and a nucleic acid sequence that varies between members of said population wherein said nucleic acid sequence that varies comprises a substrate for recombination, and wherein every member of said library has the same origin of replication. Such libraries typically comprise at least 2 different members, preferably at least 10 different members, more preferably at least 50 different members, and most preferably at least 500 different members per cell. Such libraries typically comprise at least 10⁵, preferably at least 10⁷, more preferably at least 10⁸, and most preferably at least 10⁹ up to 10¹⁵ or more different members as described above. The recombinase sites and the nucleic acid members comprise any one or more of the features described herein and may, optionally be contained within infectious particles as described herein. Thus, it will be appreciated that the libraries may comprise a collection of isolated phage, or phagemid or bacteria containing the nucleic acid library members. The nucleic acids can be phagemid vectors encoding the antibodies and ready for subcloning into a phage vector or the nucleic acids can be a collection of phagemid already carrying the subcloned antibody-encoding nucleic acids.

In still another embodiment, this invention provides methods of preparing a polypeptide. The methods involve providing a nucleic acid library as described herein; selecting one or more members of library; and expressing the nucleic acids of the selected members. In one preferred embodiment, the selecting may involve expressing proteins encoded by the members of the nucleic acid library; and screening the expressed proteins for one or more desired properties (*e.g.* specific binding to one or more preselected targets, a minimum binding avidity for one or more preselected targets, a maximum binding avidity for one or more preselected targets, thermostability at a particular preselected temperature, a predefined catalytic activity, a predefined enzymatic activity under selected conditions, a predefined biological activity; stability and/or activity under oxidizing or reducing conditions or in non-aqueous solvents, *etc.)* and selecting the library members that meet the screening criteria. In particularly preferred embodiments the screening comprises screening for specific binding to a preselected target and in such instances, particular preferred library members express antibodies (*e.g.* on replicable genetic display packages (rgdps) or on the surface of cells infected or transfected with the members of the nucleic acid library). Preferred rgdps include phage or phagemid expressing one or more polypeptides substituted for or covalently linked to surface proteins. The selected members can be used for another round of recombination and screening or to enrich or generate a library for subsequent rounds of recombination and screening.

In various other embodiments, this invention also provides host cells comprising a nucleic acid library as described herein.

### Definitions

As used herein, an "antibody" refers to a protein consisting of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes,

IgG, IgM, IgA, IgD and IgE, respectively.

A typical immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist as intact immunoglobulins or as a number of well characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region thereby converting the (Fab')₂ dimer into a Fab' monomer. The Fab' monomer is essentially a Fab with part of the hinge region *(see, Fundamental Immunology,* W.E. Paul, ed., Raven Press, N.Y. (1993), for a more detailed description of other antibody fragments). While various antibody fragments are defined in terms of the digestion of an intact antibody, one of skill will appreciate that such Fab' fragments may be synthesized *de novo* either chemically or by utilizing recombinant DNA methodology. Thus, the term antibody, as used herein also includes antibody fragments either produced by the modification of whole antibodies or synthesized *de novo* using recombinant DNA methodologies. Preferred antibodies include single chain antibodies (antibodies that exist as a single polypeptide chain), more preferably single chain Fv antibodies (sFv or scFv) in which a variable heavy and a variable light chain are joined together (directly or through a peptide linker) to form a continuous polypeptide. The single chain Fv antibody is a covalently linked V_{H}-V_{L} heterodimer which may be expressed from a nucleic acid including V_{H}- and V_{L}- encoding sequences either joined directly or joined by a peptide-encoding linker. Huston, *et al.* (1988) *Proc. Nat. Acad. Sci. USA, 85:* 5879-5883. While the V_{H} and V_{L} are connected to each as a single polypeptide chain, the V_{H} and V_{L} domains associate non-covalently. The first functional antibody molecules to be expressed on the surface of filamentous phage were single-chain Fv's (scFv), however, alternative expression strategies have also been successful. For example Fab molecules can be displayed on phage if one of the chains (heavy or light) is fused to g3 capsid protein and the complementary chain exported to the periplasm as a soluble molecule. The two chains can be encoded on the same or on different replicons; the important point is that the two antibody chains in each Fab molecule assemble post-translationally and the dimer is incorporated into the phage particle via linkage of one of the chains to, *e.g.,* g3p *(see, e.g.,* U.S. Patent No: 5733743). The scFv antibodies and a number of other structures converting the naturally aggregated, but chemically separated light and heavy polypeptide chains from an antibody V region into a molecule that folds into a three dimensional structure substantially similar to the structure of an antigen-binding site are known to those of skill in the art *(see e.g.,* U.S. Patent Nos. 5,091,513, 5,132,405, and 4,956,778). Particularly preferred antibodies should include all that have been displayed on phage (*e.g.*, scFv, Fv, Fab and disulfide linked Fv (Reiter *et al.* (1995) *Protein Eng.* 8: 1323-1331).

An "antigen-binding site" or "binding portion" refers to the part of an immunoglobulin molecule that participates in antigen binding. The antigen binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions" or "FRs". Thus, the term "FR" refers to amino acid sequences which are naturally found between and adjacent to hypervariable regions in immunoglobulins. In an antibody molecule, the three hypervariable regions of a light chain and the three hypervariable regions of a heavy chain are disposed relative to each other in three dimensional space to form an antigen binding "surface". This surface mediates recognition and binding of the target antigen. The three hypervariable regions of each of the heavy and light chains are referred to as "complementarity determining regions" or "CDRs" and are characterized, for example by Kabat *et al.* Sequences of proteins of immunological interest, 4th ed. U.S. Dept. Health and Human Services, Public Health Services, Bethesda, MD (1987).

As used herein, the terms "immunological binding" and "immunological binding properties" refer to the non-covalent interactions of the type which occur between an immunoglobulin molecule and an antigen for which the immunoglobulin is specific. The strength or affinity of immunological binding interactions can be expressed in terms of the dissociation constant (K_{d}) of the interaction, wherein a smaller Kd represents a greater affinity. Immunological binding properties of selected polypeptides can be quantified using methods well known in the art. One such method entails measuring the rates of antigen-binding site/antigen complex formation and dissociation, wherein those rates depend on the concentrations of the complex partners, the affinity of the interaction, and on geometric parameters that equally influence the rate in both directions. Thus, both the "on rate constant" (kₒₙ) and the "off rate constant" (k_{off}) can be determined by calculation of the concentrations and the actual rates of association and dissociation. The ratio of k_{off}/kₒₙ enables cancellation of all parameters not related to affinity and is thus equal to the dissociation constant K_{d} *(see, generally,* Davies *et al.* (1990) *Ann. Rev. Biochem.,* 59: 439-473.

The phrase "specifically binds to a protein" or "specifically immunoreactive with", when referring to an antibody refers to a binding reaction which is determinative of the presence of the protein in the presence of a heterogeneous population of proteins and other biologics. Thus, under designated immunoassay conditions, the specified antibodies bind to a particular protein and do not bind in a significant amount to other proteins present in the sample. Specific binding to a protein under such conditions may require an antibody that is selected for its specificity for a particular protein. For example, F5 or C1 antibodies can be raised to the c-erbB-2 protein that bind c-erbB-2 and not to other proteins present in a tissue sample. A variety of immunoassay formats may be used to select antibodies specifically immunoreactive with a particular protein. For example, solid-phase ELISA immunoassays are routinely used to select monoclonal antibodies specifically immunoreactive with a protein. See Harlow and Lane (1988) Antibodies, A Laboratory Manual, Cold Spring Harbor Publications, New York, for a description of immunoassay formats and conditions that can be used to determine specific immunoreactivity.

The terms "polypeptide", "peptide", or "protein" are used interchangeably herein to designate a linear series of amino acid residues connected one to the other by peptide bonds between the alpha-amino and carboxy groups of adjacent residues. The amino acid residues are preferably in the natural "L" isomeric form. However, residues in the "D" isomeric form can be substituted for any L-amino acid residue, as long as the desired functional property is retained by the polypeptide. In addition, the amino acids, in addition to the 20 "standard" amino acids, include modified and unusual amino acids, which include, but are not limited to those listed in 37 CFR □1.822(b)(4). Furthermore, it should be noted that a dash at the beginning or end of an amino acid residue sequence indicates either a peptide bond to a further sequence of one or more amino acid residues or a covalent bond to a carboxyl or hydroxyl end group.

The term "binding polypeptide" refers to a polypeptide that specifically binds to a target molecule (*e.g.* a cell receptor) in a manner analogous to the binding of an antibody to an antigen. Binding polypeptides are distinguished from antibodies in that binding polypeptides are not ultimately derived from immunoglobulin genes or fragments of immunoglobulin genes.

The term "nucleic acid" refers to deoxyribonucleotides or ribonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.* degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (Batzer *et al.* (1991) *Nucleic Acid Res.* 19: 5081; Ohtsuka *et al.* (1985) *J. Biol. Chem.* 260: 2605-2608; and Cassol *et al.* (1992); Rossolini *et al.,* (1994) *Mol. Cell. Probes* 8: 91-98). The term nucleic acid is used interchangeably with gene, cDNA, and mRNA encoded by a gene.

The terms "isolated" or "biologically pure" refer to material which is substantially or essentially free from components which normally accompany it as found in its native state. However, the term "isolated" is not intended refer to the components present in an electrophoretic gel or other separation medium. An isolated component is free from such separation media and in a form ready for use in another application or already in use in the new application/milieu.

The term "heterologous nucleic acid' refers to a nucleic acid that is not native to the cell in which it is found or whose ultimate origin is not the cell or cell line in which the "heterologous nucleic acid" is currently found.

The idiotype represents the highly variable antigen-binding site of an antibody and is itself immunogenic. During the generation of an antibody-mediated immune response, an individual will develop antibodies to the antigen as well as anti-idiotype antibodies, whose immunogenic binding site (idiotype) mimics the antigen. Anti-idiotypic antibodies can also be generated by immunization with an antibody, or fragment thereof.,

A "phage display library" refers to a collection of phage (*e.g.*, filamentous phage) wherein the phage express an external (typically heterologous) protein. The external protein is free to interact with (bind to) other moieties with which the phage are contacted. Each phage displaying an external protein is a "member" of the phage display library.

The term "filamentous phage" refers to a viral particle capable of displaying a heterogenous polypeptide on its surface. Although one skilled in the art will appreciate that a variety of bacteriophage may be employed in the present invention, in preferred embodiments the vector is, or is derived from, a filamentous bacteriophage, such as, for example, fl, fd, Pf1, M13, *etc.* The filamentous phage may contains a selectable marker such as tetracycline (*e.g.*, "fd-tet"). Various filamentous phage display systems are well known to those of skill in the art *(see, e.g.,* , Zacher *et al.* (1980) *Gene* 9: 127-140, Smith *et al.*(1985) S*cience* 228: 1315-1317 (1985); and Parmley and Smith (1988) *Gene* 73: 305-318).

A "viral packaging signal" is a nucleic acid sequence necessary and sufficient to direct incorporation of a nucleic acid into a viral capsid.

An assembly cell is a cell in which a nucleic acid can be packaged into a viral coat protein (capsid). Assembly cells may be infected with one or more different virus particles (*e.g.* a normal or debilitated phage and a helper phage) that individually or in combination direct packaging of a nucleic acid into a viral capsid.

The following abbreviations are used herein: AMP, ampicillin; CDR, complementarity determining region; ELISA, enzyme linked immunosorbent assay; FACS, fluorescence activated cell sorter; FR, framework region; Glu, glucose;; IMAC, immobilized metal affinity chromatography; kₒₙ, association rate constant; k_{off}, dissociation rate constant; PCR, polymerase chain reaction; RU, resonance units; scFv or sFv, single-chain Fv fragment; surface plasmon resonance; Vₖ, immunoglobulin kappa light chain variable region; V_{λ}, immunoglobulin lambda light chain variable region; V_{L}, immunoglobulin light chain variable region; V_{H}, immunoglobulin heavy chain variable region; wt, wild type.

A "recombinase" refers to a protein that mediates recombination between two or more different nucleic acids. A preferred recombinase "recognizes" one or more particular nucleotide sequences (a recombinase recognition site) and excises the nucleic acid segment at these sites. The use of a recombinase and appropriate recombinase recognition sites can thereby delineate the recombination substrate (the nucleic acid sequence subject to a recombination event).

In a preferred embodiment, an "infectious particle" refers to a nucleic acid molecule coated with 'coat proteins' which is able to enter cells at high efficiency by virtue of specific interactions between coat protein(s) and a receptor on the cell surface. An example of an infectious particle is a Ff phage or phagemid

A "coat protein" refers to a protein found on the surface of an infectious particle which 'coats' the enclosed nucleic acid. Coat proteins may be termed major of minor depending upon the number of copies present on the infectious particle. In Ff phage p8 is the major coat protein and present in approximately 2800 copies, while p3 is a minor coat protein present in 3-5 copies.

The term "infection" refers to the process by which an infectious particle enters a cell by virtue of specific interactions between a coat protein or proteins found on the surface of the infectious particle and a receptor on the cell surface. In the case of Ff phage and phagemid, the interaction occurs between p3 on the phage surface and the F pilus on the bacteria.

A "primary library" or a "primary gene library" refers to a population of individual nucleic acid molecules each of which is constituted of two parts: a nucleic acid sequence present in each molecule which is identical; and a variable nucleic acid sequence, not necessarily contiguous, which constitutes the substrate for recombination. Each nucleic acid molecule differs from the other nucleic acid molecules found in the gene library. The libraries can take a number of forms. Thus, in one embodiment the library is a collection of cells containing members of the phage display library, while in another embodiment, the library consists of a collection of isolated phage, and in still another embodiment the library consists of a library of nucleic acids encoding a phage display library. The nucleic acids can be phagemid or plasmid vectors encoding polypeptides or antibodies and ready for subcloning into a phage vector or the nucleic acids can be a collection of phagemid already carrying the subcloned polypeptide or antibody-encoding nucleic acids.

A "secondary library" or a "secondary gene library" refers to a collection of nucleic acids comprised of individual nucleic acid molecules derived from a primary gene library in which the diversity has been increased by the process of either site specific or general recombination;

A "library of infectious particles" refers to a collection of infectious particles each of which contains a nucleic acid molecule which differs from the nucleic acid molecules carried by other infectious particles in the library, such differences usually being restricted to a specific gene encoding a specific function whose properties are to be modified, all other parts of the infectious particle genome usually remaining identical. When the number of infectious particles is greater than the diversity of the library, each of these particles can be present in more than one copy.

A "recombination substrate" refers to a nucleic acid molecule which is able to recombine with another recombination substrate, either by virtue of the presence of specific recombination sites (e.g. loxP, where the recombination is catalysed by the specific recombinase, cre), or by virtue of similarities in the nucleic acid sequences which allow general cellular recombination to occur (general recombination)

The term "specific recombination" refers to recombination which occurs at specific sites (e.g. loxP) recognized by recombinases (e.g. cre recombinase) which act at such sites The term "multiplicity of infection (MOI)" refers to the ratio between the number of phage or phagemid (measured as colony forming units or plaque forming units) added to a culture of bacteria and the number of bacteria. An MOI of 200 indicates that phage(mid) are in a 200 fold excess over bacteria.

A "replicable genetic display package" or "rdgp" refers to a biological particle which has genetic information providing the particle with the ability to replicate under appropriate conditions. The particle can display on its surface at least one part of a polypeptide. The polypeptide can be encoded by genetic information native to the particle and/or artificially placed into the particle. The displayed polypeptide or a part of it, may be any member of a specific binding pair e.g. single chain Fv (scFv), heavy or light chain domains based on an immunoglobulin molecule, an enzyme or receptor etc. An rdgp may be a cell, a bacteria, a phage, a phagemid, or any other biological particle with the characteristics described above.

A vector is a nucleic acid molecule that is able to replicate itself, and any other sequence contained within it, in a host (*e.g.* bacterial) cell. Normally, vectors contain an origin of replication, an antibiotic resistance gene and restriction enzyme sites which permit the cloning of other DNA fragments.

A "display vector" refers to a vector used for the creation of rgdps. Normally, display vectors, in addition to an origin of replication and antibiotic resistance gene, contain a gene encoding a protein, within which a polypeptide can be expressed that is foreign to the biological particle, and which is displayed on the surface of the biological particle. They also preferably contain an additional origin of replication that allows the vector DNA to be incorporated within the biological particle which displays the foreign polypeptide. pDAN5, a phagemid vector, is an example of a display vector.

A "phagemid" is an infectious particle that consists of plasmid DNA containing the origin of replication of a phage (*e.g.* a filamentous phage) which is coated with phage coat proteins (*e.g.* provided by a helper phage). The term phagemid can also be used to apply to the DNA vector contained within the infectious particle, an Ff phagemid refers to a phagemid which uses an origin of replication derived from the Ff family of filamentous phages, and requires a helper phage derived from the same family Ff. pDAN5 is an example of an Ff phagemid.

A "linker" refers to an amino acid sequence that joins two other amino acid sequences. Thus, for example, a linker can join the V_{L} and the V_{H} chains of an antibody allowing them to form a correct scFv structure. In general, a linker is an amino acid sequence which covalently links two polypeptides in such a way that a single polypeptide is formed.

A "specific binding pair" or "sbp" refers to a pair of molecules (each being a member of a specific binding pair) which are naturally derived or synthetically produced, which bind to one another specifically. Examples of types of sbps include antigen-antibody, biotin-avidin, hormone-hormone receptor, receptor-ligand, enzyme-substrate, IgG-protein A.

A "phage display library" refers to a collection of phage (*e.g*., filamentous phage) wherein the phage express an external (typically heterologous) protein. The external protein is free to interact with (bind to) other moieties with which the phage are contacted. Each phage displaying an external protein is a "member" of the phage display library.

The term "filamentous phage" refers to a viral particle capable of displayimg a heterogenous polypeptide on its surface. Although one skilled in the art will appreciate that a variety of bacteriophage may be employed in the present invention, in preferred embodiments the vector is, or is derived from, a filamentous bacteriophage, such as, for example, f1, fd, Pf1, M13, *etc.* The filamentous phage may contains a selectable marker such as tetracycline (*e.g.*, "fd-tet"). Various filamentous phage display systems are well known to those of skill in the art (see, *e.g., ,* Zacher *et al.* (1980) *Gene* 9: 127-140, Smith *et al*.(1985) *Science* 228: 1315-1317 (1985); and Parmley and Smith (1988) *Gene* 73: 305-318).

A "selectable marker refers" to a nucleic acid sequence that allows organisms expressing that sequence and/or having that sequence present to be distinguished from organisms not expressing that sequence and/or not having that sequence present. Selectable markers are well known to those of skill in the art. Some examples include the hprt gene (Littlefield (1964) Science 145:709-710), the tk (thymidine kinase) gene of herpes simplex virus (Giphart-Gassler *et al.* (1989) Mutat, Res. 214:223-232), the nDtII gene (Thomas et al. (1987) Cell 51:503-512; Mansour *et al.* (1988) *Nature* 336:348_352), or other genes which confer resistance to amino acid or nucleoside analogues, or antibiotics, *etc.*

A "loxP site" refers to a recombinase recognition site that can act as a recombinse recognition site for Cre. A loxP site includes native loxP sequences as well as modified loxP sites. Modified loxP sites are well known to those of skill in the art and include, but are not limited to loxP 511, fas, and other various mutations such as have been described in the literature *(see, e.g.,* Mack *et al., supra;* Hoess *et al.* (1986), *supra;* Hoess *et al.* (1984) *Biochem"* 81: 1026-29; Hoess *et al.* (1985) *Gene,* 40: 325-329; Abremski *et al.* (1986) *J. Biolog. Chem.,* 261: 391-396; Sauer (1996) *Nuc. Acids Res.* 24: 4608-13).

### Brief description of the drawings

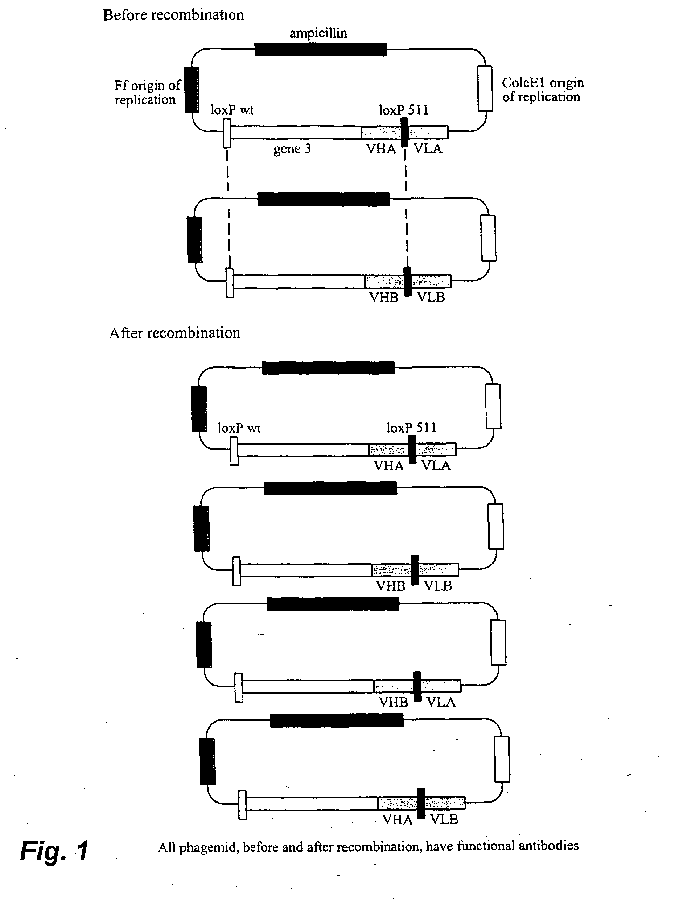

Figure 1 illustrates the generation of diversity (*e.g.* an antibody library) the one vector system in accordance with the present invention. Infection of a bacterium by two phagemids containing V_{H}A/V_{L}A and V_{H}B/V_{L}B V regions is shown. After recombination, four products will be present: the original V_{H}A/V_{L}A and V_{H}B/V_{L}B, and the two new products V_{H}A/V_{L}B and V_{H}B/VLA. All phagemid, both before and after recombination, contain functional antibodies (scFv). In addition to the number of cells used, the size of the final gene library is also determined by the number of phagemids which enter a cell and by the extent of recombination which occurs.

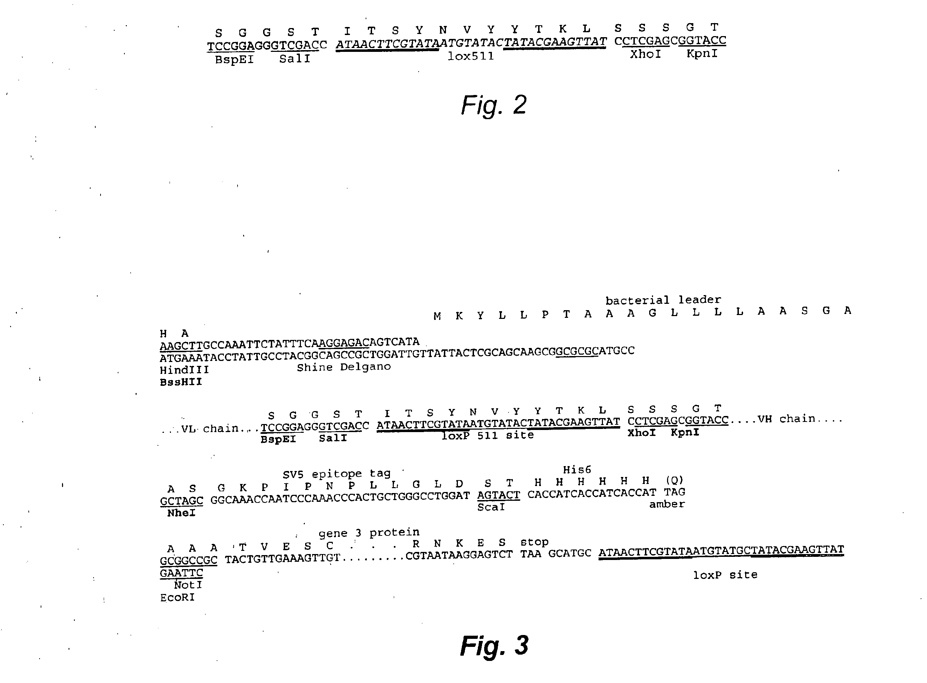

Figure 2 illustrates the sequence of the loxP 511 scFv linker used in the examples. In particular, the DNA and deduced amino acid sequences of the loxP 511 linker used to create mini-libraries and for cloning scFv is indicated. Restriction sites used for cloning V regions are indicated below the DNA sequence. The sequence of the loxP 511 recognition site, involved in recombination, is in italics, restriction sites used for cloning are underlined, and the inverted repeats in the loxP 511 recognition sequence are double underlined. Antibody V regions were cloned in the order V_{L} linker V_{H}.
Figure 3 illustrates the pDAN5 polylinker described herein. The sequence of the pDAN5 polylinker for the cloning of scFvs is given (the sequence of the V regions and gene 3 is omitted). The position of the V_{L} and V_{H} chains are given, and the restriction sites used to clone them are indicated in bold. The loxP 511 and loxP wild type sites used to induce recombination are indicated, with the inverted repeats underlined. Other important features, including the leader sequence, the SV5 tag used to recognize the scFv, the His tag for purification, the amber stop codon and gene 3 are also indicated.

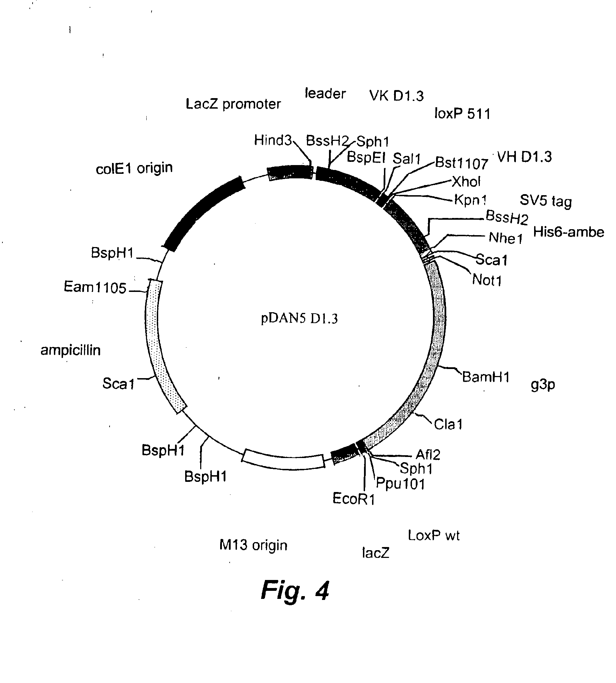

Figure 4 illustrates the map of pDAN5. In particular, a map of the display vector pDAN5 with the D1.3 scFv cloned is shown. Sites used for V gene cloning are in bold.

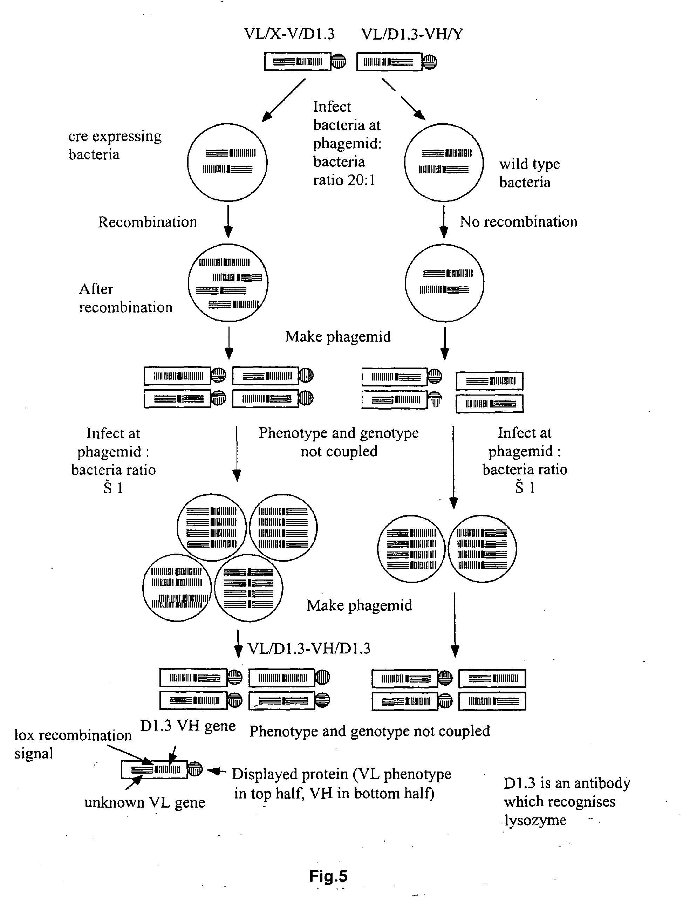

Figure 5 illustrates the scheme of the D1.3 recombination experiment Two phagemid containing V_{L}/X-V_{H}/D1.3 and VL/D1.3-VH/Y (where X and Y represent unknown antibodies) were used to infect either cre expressing bacteria or wild type DH5αF (which do not express cre recombinase) at high multiplicity of infection (MOI). After growth for about 12 hours, phagemid were made, reinfected into DH5αF (i.e. not in cre expressing cells) to couple genotype and phenotype, and tested by PCR and ELISA for the presence of functional VL/D1.3-VH/D1.3.

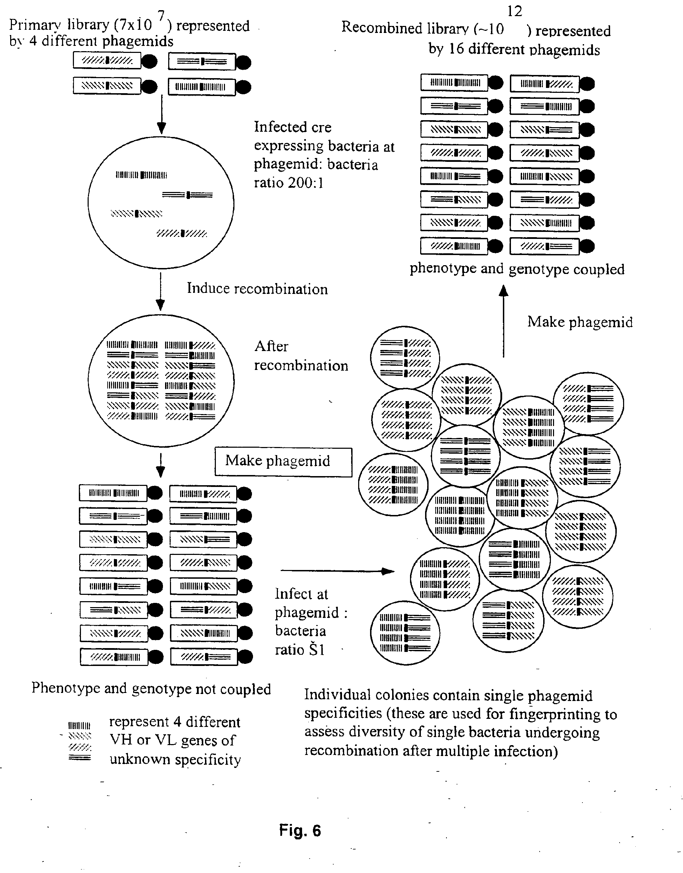

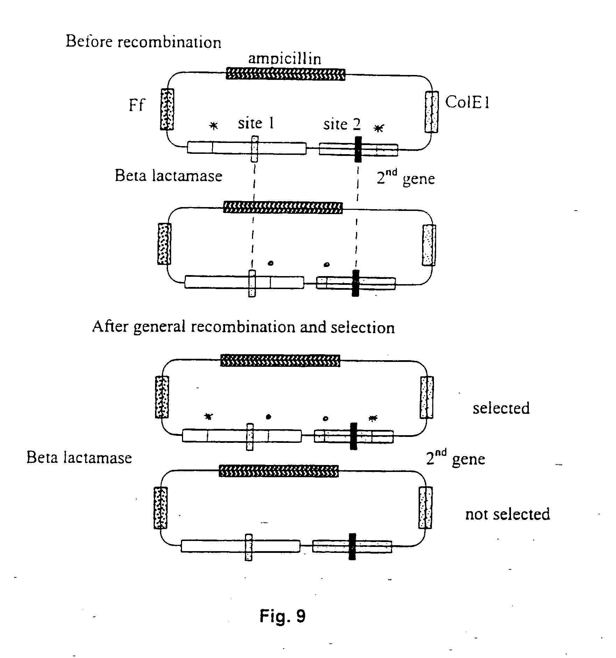

Figure 6 illustrates a scheme for the creation of a secondary gene library by in vivo recombination. Bacteria expressing cre are infected by primary library phagemid at an MOI of 200:1. After recombination, phagemid are prepared and reinfected into DH5αF at MOI less than 1 to couple phenotype and genotype. These phagemids, with phenotype and genotype coupled, are used for library selections. The diversity created from the infection of a single bacteria with only four phagemid (with different VH and VL genes shaded differently) is indicated for simplicity. However, according to the present invention, a single bacteria is infected with a far higher number of phagemids. PCR fingerprinting experiments (see figure 9) confirm these data.

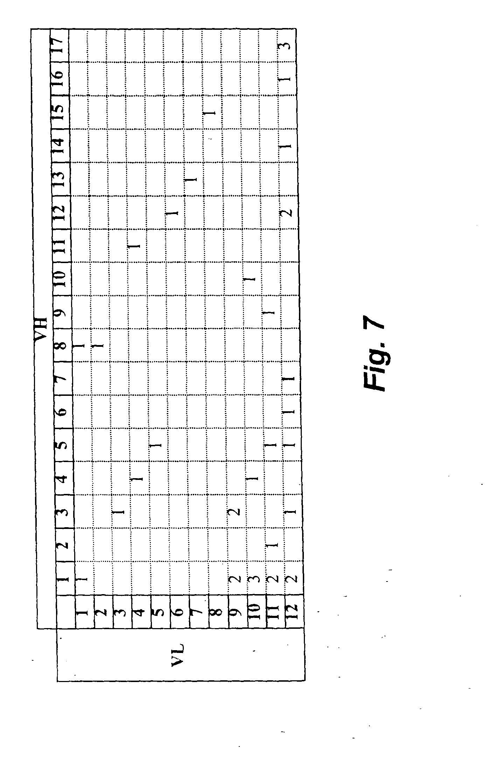

Figure 7 illustrates the evaluation of diversity in bacteria expressing cre recombinase infected by multiple phagemids. Individual bacterial colonies expressing single phagemid specificities (after reinfection at MOI less than 1) were isolated after recombination as described in figure 6. V_{H} and V_{L} genes from such colonies were amplified using VH and VL specific primers (Sblattero and Bradbury (1998) *Immunotechnology* 3: 271-278) and fingerprinted using BstNI. The different V_{H} gene fingerprints were numbered horizontally 1-17 for cell 1, and 1-18 for cell 2, while the different V_{L} fingerprints were numbered vertically 1-12 for cell 1 and 1-14 for cell 2. There is only one table, representing one cell in figure 7. The V_{H} and V_{L} fingerprints found in 37 individual bacteria for cell 1 and 41 individual bacteria for cell 2 were analyzed and are summarized in the table, with the number of times each VH/VL combination was found being indicated. This data give an indication of the number and different combinations of VH/VL present in the original cells 1 and 2.

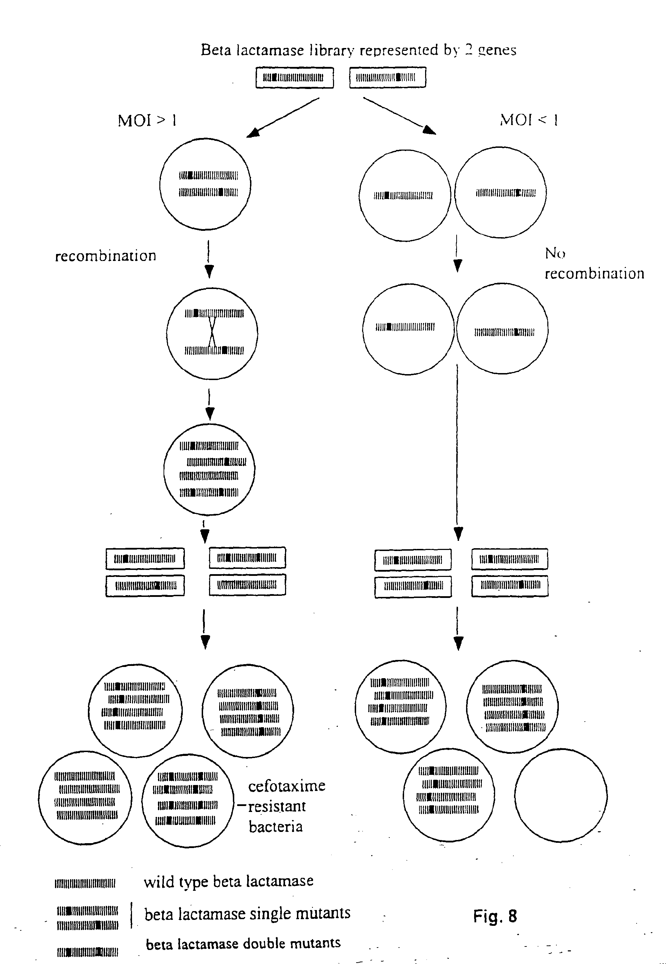

Figure 8 illustrates the scheme of general recombination used to create cefotaxime resistant genes. Wild type bacteria were infected by a beta lactamase primary phagemid library at an MOI of greater than 10:1. Recombination occurs, bringing mutations which were initially in different genes into the same gene. Only phagemid containing the recombined beta lactamase genes are able to confer resistance to cefotaxime and will be selected. The primary library used had a diversity of 5x10⁵. This is represented in the figure, by two phagemid, each of which contains a single mutation indicated by an asterisk or by a point. Neither of these mutations alone is able to confer resistance to cefotaxime, whereas both together (arising after recombination) can confer resistance to cefotaxime. The phages selected for the recombination event occurred on the beta lactamase gene will carry also a new combination of mutation on the second gene.
Figure 9 illustrates scheme of general recombination which may be used to select for recombined genes by linking them to recombination of a marker or selector gene. A library of different genes, or mutant versions of a single gene, would be cloned into a vector which itself is a library of vectors containing mutations within a marker or selector gene (*e.g.* beta lactamase). Such a library could also be prescreened to contain only known beneficial mutations in order to increase the incidence of useful recombination events, or could even be made up of two single mutations which are effective only when found on the same gene. Upon selection on the selection medium (*e.g.* cefotaxime if beta lactamase is being used) only those vectors which have undergone recombination within the beta lactamase gene will be selected for. As recombination usually occurs in two sites, this will simultaneously select for vectors which have undergone recombination in another part of the vector, which frequently will be within the gene to be recombined. The figure illustrates the situation with two vectors, each of which contain beneficial mutations within both the selector gene (beta lactamase) and the gene of interest. Recombination within the beta lactamase gene, mediated by the cellular recombinase machinery, brings the two beta lactamase mutations in cis and so permits selection by cefotaxime resistance. Simultaneously, there is recombination within the gene of interest which also brings to beneficial mutations in cis and permits identification of this useful gene by selection or screening. In the scheme open boxes represent phagemids and open circles represent the vector in its replicative (plasmidic) form.

### Detailed description of the invention

This invention provides novel methods to create large, highly diverse libraries of nucleic acids and/or expressed proteins. The creation of diversity in populations of nucleic acids or polypeptides has become an important tool in the derivation of polypeptides with useful characteristics. Such approaches typically involve producing a large diverse population of subject molecules and then screening those molecules for the desired property or properties of interest. Such methods thus mimic the processes of evolution and natural selection and can be used to evolve and select molecules having a wide variety of desired properties.

Such selection methods have been widely used in a variety of contexts. Thus, for example, directed evolution and selection procedures have been utilized to obtain single chain human, non-human or chimeric antibodies having a particular binding specificity and/or avidity *(see, e.g.,* Stemmer et al. (1992) *Biotechniques* 14:256-265; Marks, Marks *et al.* (1992) *Bio*/*Technology,* 10: 779-782, *etc.).* Such approaches have also bee used to obtain particular binding proteins *(see, e.g.,* Clackson and Wells (1994) *Trends in Biotechnology* 12: 173-184), to obtain RNA enzymes (Beaudry *et.al.* (1992) 257: 635-641), to evolve and select proteins having particular enzymatic or catalytic activities.

Unlike prior art approaches that utilize site-directed or random mutagenesis approaches (*e.g.* error-prone PCR) to increase the diversity of a library, the methods of this invention utilize recombination processes. These include the natural (endogenous) recombination mechanisms that occur within a cell (*e.g.* crossing-over, homologous recombination, *etc.)* or recombinase-mediated processes where the process is mediated by an endogenous and/or exogenous recombinase.

Unlike other approaches that exploit recombinases to mediate recombination between two different constructs (*e.g.* a vector and a genomic DNA, two different vectors, *etc.*), in one embodiment, the methods of this invention utilize recombinases to mediate recombination between two or more identical constructs; that is constructs that are essentially identical but for the segment(s) that are to be recombined.

It was surprising discovery of this invention that, contrary to the teachings in the prior art, multiple constructs of the same class (*e.g*. phagemid, plasmid, *etc*.) that are essentially identical in regulatory elements (*e.g.* origin of replication and/or promoter(s) and/or enhancers and/or termination sequences) and/or selectable markers (*e.g*. antibiotic resistance genes) can co-exist in a host cell (*e.g*. a bacterial cell) for a sufficiently long time that extensive recombination (*e.g*., mediated by non-recombinase cellular mechanisms, by cellular recombinases, or by exogenous recombinases) can occur between the constructs and permit the creation of large highly-diverse nucleic acid and/or peptide libraries. Moreover, it was also a discovery of this invention that recombination is particularly enhanced in cells infected at high multiplicity of infection (*e.g.* m.o.i. greater than about 5, more preferably m.o.i. greater than about , and most preferably m.o.i. greater than about 200 or more). It was also a finding of this invention that libraries cloned in vectors with high copy numbers (*e.g.* greater than 300) have a longer co-permanence than vectors with low copy numbers (*e.g.* 10) allowing recombination to occur more extensively. Thus, transfection to introduce more than one copy of vector (preferably 200, 300, or even greater than 500) into each cell should result in recombination.

Because recombination takes place in a single class of essentially identical (but for the nucleic acid(s) being recombined) constructs the problems associated with the so-called two-construct systems described above are overcome. Thus, for example, all of the constructs introduced into a single cell according to the methods of this invention can have identical origins of replication, and/or selectable markers, and/or control sequences. Thus, only a single type of "recombination vector" need be constructed and the various sequences to be recombined can be inserted into the vector at one or more preselected sites (*e.g.* provided by one or more polylinkers). Moreover there can be as many as 500, 1000 or more constructs inside a cell, limited only by the copy number of the vector used, and recombination can occur between any or all of these constructs. Thus an extremely high degree of diversity can be produced in a single cell and a modestly sized population of cells *(e.g.* 10⁵-10⁷ cells) can produce a library of remarkable diversity (*e.g.* 10¹¹-10¹⁸ or more different members). The system is not constrained to a single recombinant per cell.

In addition, even after recombination, the constructs used in the methods of this invention are essentially unchanged, but for the recombination. Consequently they can be used to increase diversity in other libraries or in multiple rounds of recombination and selection with essentially no modification.

These advantages are merely illustrative. Other properties and advantages will be apparent to one of skill in light of the present description.

The libraries, once produced, can be used to modify or improve particular properties. In any recombined library a small proportion of proteins (*e.g.* scFv) will have particular properties, such as good folding, resistance to reducing agents or high temperature. By preeselecting for any of these properties, a small subset of the recombined library will be obtained which is enriched in that property. The power of the recombination method described here allows the creation of new libraries which may be as large as the initial recombined library, but which are biased towards the property which has been preselected for. For example, in the case of scFv libraries, selection of the recombined library on protein LA (which recognises a conformational epitope on some V_{H} and V_{L} regions) preselects for well folded scFv not susceptible to degradation. Such scFv are present in a small proportion of the final recombined library, but by performing further rounds of recombination, a very diverse scFv library composed of well folded scFv can be created. Similarly, a small proportion of scFv in the recombined library may be resistant to reducing agents such as DTT. By treating the library with DTT and selecting those scFv which survive (e.g. by the selection of a tag located at the N terminus, or by using protein LA), scFv which are resistant to this agent can be selected. A further round of recombination will create a new library which is enriched in scFv which are resistant to DTT, and so may be functional intracellularly. Phage(mid) antibody libraries are usually grown at low temperatures (*e.g.* 30°C). This is because many antibodies are toxic or unstable at higher temperatures. By growing bacteria of the recombined library at higher temperatures (*e.g.* 37°C or even 42°C) only those bacteria containing less toxic and more stable scFv will be selected. By performing another round of selection, all scFv in the library will be made up of those V_{H} and V_{L} regions which are preselected for non-toxicity or stability. Similarly, by selecting for binding to a particular target, a restricted library directed towards a particular specificity will be derived. By carrying out recombination among these scFv, all of which recognize a particular antigen, higher affinity antibodies which were not present in the original library can be selected. Similar methods of preselection, for example resistance to particular solvents, ionic conditions, temperatures, activity etc. can be envisaged.

### I. Efficient creation of a large highly diverse nucleic acid library.

In one preferred embodiment, this invention provides methods of making a large highly diverse nucleic acid library. The methods involve transfecting at least one cell, (more preferably a population of cells), with more than one nucleic acid molecule per cell (preferably greater than 2, more preferably greater than 10 and most preferably more than 50 or 500). Where an infective particle is used, the cell (or population of cells) is preferably infected at a multiplicity of infection greater than 1 (e.g. m.o.i. greater than 5, more preferably m.o.i. greater than 20, and most preferably m.o.i. of about 200 or more).In preferred embodiments, the nucleic acid molecules are members of a library in which each nucleic acid molecule has the same (identical) origin of replication and/or selectable markers (*e.g.* antibiotic resistance gene(s)), and or regulatory sequences. Each of the nucleic acid molecules also contains a substrate for recombination which is different in each nucleic acid molecule.

The method is easily understood by reference to Figure 1. For the purposes of simplicity, recombination between only two phagemid has been shown schematically, but it is clear that the number of different vectors which can infect a single cell is very high.

The substrates for recombination are cloned into a single vector backbone, in order to constitute a phagemid containing the origins of replication ColEl and Ff. Selectable markers (*e.g.*, beta lactamase or antibiotic resistance gene(s)) are optionally present. Two phagemids carrying the genes for V_{H}A/V_{L}A and V_{H}B/V_{L}B respectively, are created. Following recombination, 4 different phagemids are obtained: the two starting phagemids (V_{H}A/V_{L}A and V_{H}B/V_{L}B) and two new phagemids (V_{H}A/V_{L}B and V_{H}B/V_{L}A).

As already stated above, a simplified scheme has been shown in Figure 1. In fact, a single bacterial cell can be infected by a very high number of vectors. Thus, in example 3, a single cell is infected by up to 19 phagemid. This results in a recombinatorial potential which is enormous. In example 3, a single cell can contain up to 361 (19 x 19) different library members. Similarly, a cell containing 50 constructs could, after recombination, contain as many as 2500 different library members.

The resulting library members can be subjected to one or more rounds of screening whereby the library is enriched for population members expressing certain desired properties. This enriched library can be combined with another library to increase the diversity of that library or it can itself be subject to another round of recombination in host cells as described above. The process of selection and recombination of the selected library members can be repeated as many times as desired until a library containing members exhibiting the desired properties is obtained.

It will be noted that where the library members are capable of being packaged (*e.g.* in a replicable display package) it is possible that the particles thus produced may have a genotype that is de-coupled from the phenotype of the package. Thus, for example, where the library members contain a substrate for recombination that expresses a protein on the surface of a phage (*e.g.* a pIII coat protein), because the cell contains many different library members and the packaging machinery is "independent" of the nucleic acid sequence comprising the library member, it is possible that the coat protein in which library member (nucleic acid is packaged) is not the coat protein encoded by that nucleic acid. In other words, the phenotype (coat protein) of the package is "decoupled" from the genotype (nucleic acid) of the package.

Because the library members of this invention are readily amendable to re-use in further rounds of recombination/selection, the genotype and phenotype of the replicable display package can be readily re-coupled (*e.g.* as illustrated in Figure 6). To accomplish this the nucleic acid library, or a fraction thereof, is used to retransfect/reinfect host cells at a low m.o.i. or low copy number (*i.e.*, copy number or m.o.i. of about 1). This produces a second "derivative" library having, on average, one library member per cell. In this instance, when the nucleic acid members are re-packaged, since there is only one library member (although possibly many copies of that one library member) per cell, essentially all the replicable genetic package coat proteins (*e.g*. phagemid coat proteins) not provided by a helper cell are encoded by the library member. When the nucleic acid library member is packaged it will be packaged into a protein coat comprising proteins encoded by that library member. In this instance, the phenotype of the replicable display package is encoded by the nucleic acid contained within that package. In other words, genotype and phenotype of the package are linked.

The recombination described above can take place by exploiting the non-recombinase recombination systems present in the cell, and in this case it is referred to as general recombination, or it can take place at specific sites (*e.g.* be recombinase mediated). The efficiency of general recombination can be increased using bacterial strains containing certain mutations, for example, mutS or recO98 or with physical treatments, for example UV treatment, chemical mutagenesis, *etc*.

Typically, general mutation takes place a much lower efficiency than recombinase-mediated recombination. Thus, in preferred embodiments utilizing general recombination, it is often desirable to include a selection step to positive select for recombinants before subsequent screening and/or recombination steps. Various selection strategies are known to those of skill in the art. However, one particularly preferred selection strategy is illustrated in Example 4 and in Figure 9. Briefly one or more selectable markers are coupled to the segments it is desired to recombine. Preferred selectable markers are chosen that are defective in the absence of a recombination event. When recombination occurs, the marker's selectable property is restored and the recombined member can be isolated. In parallel, in a high proportion of cases, recombination will also occur in the segments which it is desired to recombine. Thus, by way of illustration, in Example 4, wild type beta lactamase which is normally is unable to provide significant resistance to the antibiotic cefotaxime becomes a significant cefotaxime resistance marker when recombination events occur and thereby provides an effective marker for selecting recombinants.

Where recombinase-mediated recombination is utilized, recombination typically takes place at least one, more preferably at least two or more recombinase recognition sites. Typically the recombinase recognition sites will be engineered into the nucleic acid library member such that they flank one or more sequences that are recombination substrates *(i.e.* sequences that it is desired to recombine). Thus, for example, the in the case of an antibody library, the recombinase recognition sites will be placed such that they flank one or more antibody regions (*e.g.*, a variable heavy chain, a variable light chain), *etc.* It is possible to provide many different recombinase recognition sites in a single construct and these can be sites for one type of recombinase or multiple recombinase systems can be used.

The recombinase mediated recombination can be mediated by an endogenous recombinase in the cell and/or by an exogenous recombinase. The exogenous recombinase can be an exogenous recombinase protein delivered to the cell, but in a more preferred embodiment the exogenous recombinase is expressed by a nucleic acid that has been transfected into the cell. The cell can also be a cell that has been modified to express an endogenous recombinase at an abnormally high level.

### II. Constructs and host cells for the creation of diverse libraries.

### A) Host cells.

Virtually any cell capable of being transfected or infected with the nucleic acid members of the libraries described herein and capable of supporting recombination (general or recombinase-mediated) can be used in this invention. Eukaryotic cells, including yeast, plant, fungal, insect, and mammal cells can be used, as well as both gram negative and gram positive prokaryotic cells, that can be infected by infectious particles that normally infect such cells, or have been designed or selected to infect such cells. The cells used may be normal, mutated to induce higher levels of recombination, or engineered to express specific recombinases capable of acting at specific nucleic acid sequences present within the nucleic acid molecules contained in the infectious particles. Particularly preferred cells include bacterial cells *(e.g. E. coli),* yeast cells, and mammalian cells.

### B) Vectors for use in the invention.

The nucleic acid members of the libraries described herein, can be in virtually any convenient form, with preferred forms being forms that facilitate replication and cloning. Thus, while in some embodiments, the nucleic acid library members can simply be recombination sites with associated recombinase recognition sites, preferred nucleic acid library members are vectors including, but not limited to plasmids, cosmids, and phagemids, filamentous phage (M13, F1, fd, *etc),* and the like. While not preferred, certain embodiments, particularly those that utilize large nucleic acid segments, may utilize P1 vectors, YACs, and BACs.

Thus, as indicated above, the use of invention is not limited to E. *coli* and phagemids. For 5 methods utilizing infections vectors, any infectious particle which is in a position to infect a cell such that multiple copies (*i.e*., two or more) of the nucleic acid can enter the cell and become substrates for recombination is suitable. In one preferred embodiment, an infectious particle can be considered to be a nucleic acid molecule covered with "coat molecules" (*e.g.* proteins) that enable the particle to enter the cells with high efficiency, *e.g.*, by virtue of specific interactions between the coat molecules and a receptor or marker on the cellular surface. Such infectious particles may be naturally occurring, engineered to contain recombination substrates, or recombinant infectious particles which lack the necessary genetic information to replicate themselves, but are able to enter cells specifically, and preferably at high efficiency. A phagemid is an example of such an infectious particle.

In preferred embodiments, the members of the nucleic acid libraries of this invention have nucleic acid sequences (domains) that are identical for essentially every member of the library and other sequences that vary between members of the library. When it is said that the sequences are identical for "every" member of the library, it will be appreciated that recombinant DNA methodologies are not perfect and a certain fraction of the library will contain defective and/or contaminating members in which the "identical" sequences differ. These "abnormal" members will preferably occur at low frequency so that typically at least 80%, preferably at least 90%, more preferably at least 95%, and most preferably at least 99% or at least 99.9% of the members of the library have identical sequences.

The "identical" sequences are typically longer than a codon and in preferred embodiments are at least 10, preferably at least 20, more preferably at least 50 contiguous nucleotides in length. The identical sequences typically include at least one origin of replication and/or one or more selectable markers (*e.g.* an ampicillin resistance gene). Other features optionally present in the "identical" sequences include, but are not limited to a secretory leader, tags (*e.g.*, SV5 and His), a gene or cDNA encoding a phage or bacterial coat protein (*e.g.* gene3), PCR primer sites, recombinase recognition sites (*e.g.*, loxP, loxP511, *etc.),* vector "backbone", and the like.

In certain preferred embodiments, the "identical" sequences include essentially every sequence other than the sequences(s) that it is desired to recombine and the "identical" sequences are preferably sequences of the same "type" of molecule (*e.g.* phage, plasmid, phagemid, *etc*.). Thus, the "library" members may be viewed as identicall constructs into which the "variable" (recombination) substrates are inserted.

The "recombination substrate" sequences will vary between members of the library. It is not necessary that every member of the library differ from every other member of the library. However to the extent a library is large and diverse the probability of selecting a molecule having the desired properties increases. One may view the library as having, on average X members of which y% are different (*e.g.* a library having 10¹² member of which 90% are different will contain about 10¹¹ different members) or alternatively measure the library size as the average number of different members. Thus a library having 10¹² physical members 90% of which are different may be regarded as a library of about 10¹¹ members.

The recombination substrate can be a single sequence or two or more sequences. In particularly preferred embodiments, the recombination substrates include at least two sequences. The sequences can be contiguous or can contain intervening sequences (*e.g.* linkers and/or recombinase recognition sites, *etc.).*

As indicated above, the "identical" sequences can include one or more recombinase recognition sites. It is noted that when recombination occurs (*e.g.* in the Cre-lox system) often the recombinase recognition site is cut and a portion of that site moves with the excised nucleic acid. The site is effectively reconstituted when the fragment is re-inserted in the target DNA so that effectively the recombinase recognition site remains unchanged and is regard as "identical".

A number of suitable recombination sites, including, but not limited to, loxP, loxP mutants, and the like are described below.

The recombination substrate may include one or more genes or cDNAs encoding one or more polypeptides, or they may be nucleic acid sequences with biological activity themselves, such as sequences involved in genetic regulation, sequences encoding ribozymes, DNA enzymes and the like.

One particularly preferred library member is illustrated by the vector pDAN5 (example 2). The vector pDAN5 consists of the origin of replication, the ampicillin resistance, a secretory leader, the tags (SV5 and His), gene3 and the Ff origin of replication. In the example provided, there are two "variable" sequences that encode respectively a V_{H} and a V_{L} region of a single chain antibody. A linker comprising a recombinase recognition site (*e.g.* loxP) is between the V_{H} and V_{L} segments. This construct is not limited to the particular V_{H} and V_{L} illustrated in the example, but virtually any other V_{H} and/or V_{L} can be substituted. In addition, recombinase recognition sites need not flank simply a VH or VL, but instead can flank any desired antibody fragment or combination of fragments. In addition multiple recombinase recognition sites can be provided and they can be selected to work with the same or multiple different recombinases.

As indicated above, in certain preferred embodiments, the members of the nucleic acid library are designed so that they are capable of infecting a host cell. Typically such nucleic acid members are packaged in infectious particles and, in such instances, the "identical" (invarying) component of the member preferably encodes one or more sequences essential for packaging and/or replication and/or survival within the cell. The invarying components of such a vector can provide all the nucleic acid sequences necessary and sufficient for the packaging replication and survival in the host cell or they can be selected such that replication packaging and survival are carried out with the aid of a helper construct (*e.g.* a helper plasmid).

### C) Display libraries.

The members of the nucleic acid libraries of this invention can be designed so that when they are packaged into a replicable genetic package (*e.g.* phage, bacteria, *etc.)* they display an encoded protein on the surface of the package. Where the encoded protein is one that is encoded by one or more recombination substrates the collection of packages provides a convenient system "displaying" the vast numbers of recombined sequences available. The displayed proteins can be subject to one or more screening/selection steps (*e.g.* for binding specificity/avidity, catalytic activity, *etc.)* and selected library members can be used to generate subsequent libraries for subsequent rounds of enrichment/selection. As explained below, the "display" library can be monovalent or polyvalent.

### 1) Mono-valent antibody libraries and polypeptide libraries.

The ability to express polypeptide and antibody fragments on the surface of viruses which infect bacteria (bacteriophage or phage) or bacteria *(see, e.g.,* Charbit, *et al.* (1988) *Gene,* 70(1): 181-189) makes it possible to isolate a single binding polypeptide or antibody fragment from a library of greater than 10¹⁰ nonbinding clones. To express polypeptide or antibody fragments on the surface of phage (phage display), a polypeptide or an antibody fragment gene is inserted into the gene encoding a phage surface protein (*e.g.*, pIII) and the antibody fragment-pIII fusion protein is displayed on the phage surface (McCafferty *et al.* (1990) *Nature,* 348:552-554; Hoogenboom *et al.* (1991) *Nucleic Acids Res.* 19: 4133-4137). Since the antibody fragments on the surface of the phage are functional, phage bearing antigen binding polypeptides or antibody fragments can be separated from nonbinding phage by antigen affinity chromatography (McCafferty *et al.* (1990) *Nature,* 348: 552-554). Depending on the affinity of the antibody fragment, enrichment factors of 20 fold - 1,000,000 fold are obtained for a single round of affinity selection. By infecting bacteria with the eluted phage, however, more phage can be grown and subjected to another round of selection. In this way, an enrichment of 1000 fold in one round can become 1,000,000 fold in two rounds of selection (McCafferty *et al.* (1990) *Nature,* 348: 552-554). Thus even when enrichments are low (Marks *et al.* (1991) *J. Mol. Biol.* 222: 581-597), multiple rounds of affinity selection can lead to the isolation of rare phage. Since selection of the phage antibody library on antigen results in enrichment, the majority of clones bind antigen after four rounds of selection. Thus only a relatively small number of clones (several hundred) need to be analyzed for binding to antigen. Typically such phage-display libraries when used to display antibodies are monovalent displaying a single antibody on their surface of each library member.

### 2) Polyvalent antibody libraries

Unlike the multivalently displayed peptide phage libraries, phage antibody libraries typically display monomeric single chain Fv (scFv) or Fab antibody fragments fused to pIII as single copies on the phage surface using a phagemid system (Marks *et al.* (1991) *J. Mol. Biol.* 222: 581-597; Sheets *et al.* (1998) *Proc. Natl. Acad. Sci. USA* 95: 6157-6162.). As used herein, a polyvalent phage display antibody library, refers to a library in which each member (*e.g.* phage particle) displays, on average) two or more binding domains, wherein each binding domain includes a variable heavy and a variable light region. More generally, a multivalent phage display library displays, on average, two or more pIII fusions per page particle. Polyvalent phage display can be achieved by expressing diabodies (*i.e.*, a protein formed by fusion or conjugation of two single chain antibodies (*e.g.* scFv)) or by display of, on average, two or more antibodies on each phage particle. In contrast, a mono-valent library displays, on average, one single-chain antibody per viral particle.

### i) Diabody expression.

Diabodies are scFv dimers where each chain consists of heavy (V_{H}) and light (V_{L}) chain variable domains connected using a linker (*e.g.* a peptide linker) that is too short to permit pairing between domains on the same chain. Consequently, pairing occurs between complementary domains of two different chains, creating a stable noncovalent dimer with two binding sites (Holliger *et al.* (1993) *Proc. Natl. Acad. Sci.* 90: 6444-6448). In one approach, particular diabody genes are subcloned for expression as pIII fusions in the phagemid *(see, e.g.,* Hoogenboom *et al.* (1991) *Nucleic Acids Res.* 19: 4133-4137). This yields phagemid predominantly expressing a single scFv or diabody-pIII fusion after rescue with helper phage (Marks *et al.* (1992) *J. Biol. Chem.* 267: 16007-16010). Diabody phagemid display a bivalent antibody fragment resulting from intermolecular pairing of one scFv-pIII fusion molecule and one native scFv molecule. Using the teachings provided herein one of skill in the art can routinely produce other diabodies.

### ii) Polyvalent display of single-chain antibodies.

As an alternative to the use of diabodies, antibody phage display libraries are created in which each viral particle, on average, expresses at least 2, preferably at least 3, more preferably at least 4, and most preferably at least 5 copies of a single chain antibody.

In principle, each copy of pIII on the phage (and there is controversy as to whether there are 3 or 5 copies of pIII per phage) should express an antibody. However, proteolysis occurs and the number actually displayed is typically less. Thus, preferred multivalent antibody libraries are constructed in a phage vector and not a phagemid vector. This means that helper phage need not be added to make phage. Helper phage bring into the E. *coli* wild-type pIII that competes with the scFv-pIII fusion. Thus, in phagemid vector, this competition leaves, on average, to only 1 (or less) antibody per phage.

To produce multivalent antibody libraries, the single chain antibodies, typically expressed in phagemid, are subcloned from the phagemid vector into a phage vector. No helper phage is required and there is no competition between the wild-type pIII and the fusion scFv pIII fusion. Thus, on average, the phage display two or more pIII fusions.

### IV. Recombinase systems for use in this invention.

### A) Various recombinases.

In the present invention, the exchange of nucleic acid segments is achieved by the use of recombination proteins, including recombinases and associated co-factors and proteins. Various recombination proteins are described in the art. Examples of such recombinases include, but are not limited to the Cre family, the Integrase family, and the resolvase family.

Cre, a protein from bacteriophage P1 (Abremski and Hoess (1984) *J. Biol. Chem*.259(3):1509-1514) catalyzes the exchange (*i.e.*, causes recombination) between 34 bp DNA sequences called FoxP (locus of crossover) sites *(see, e.g.,* Hoess *et al,* (1986) *Nucl. Acids Res.* 14(5): 2287). Cre is available commercially (Novagen, Catalog No. 69247-1).

Recombination mediated by Cre is freely reversible. Cre works in simple buffers with either magnesium or spermidine as a cofactor, as is well known in the art.- The DNA substrates can be either linear or supercoiled. A number of mutant loxP sites have been described (Hoess *et al., supra*)*.* One of these, IoxP 511, recombines with another IoxP 511 site, but will not recombine with a IoxP site.

Integrase is a protein from bacteriophage lambda that mediates the integration of the lambda genome into the E. *coli* chromosome. The bacteriophage lambda Int recombinational proteins promote irreversible recombination between its substrate att sites as part of the formation or induction of a lysogenic state. Reversibility of the recombination reactions results from two independent pathways for integrative and excisive recombination. Each pathway uses a unique, but overlapping, set of the 15 protein binding sites that comprise att site DNAs. Cooperative and competitive interactions involving four proteins (Int, Xis, IHF and FIS) determine the direction of recombination. Integrative recombination involves the Int and IHF proteins and sites attP (240 bp) and attB (25 bp). Recombination results in the formation of two new sites: attL and attR. Excisive recombination requires Int, IHF, and Xis, and sites attL and attR to generate attP and aftB. Under certain conditions, FIS stimulates excisive recombination. In addition to these normal reactions, it should be appreciated that attP and attB, when placed on the same molecule, can promote excisive recombination to generate two excision products, one with attL and one with attR. Similarly, intermolecular recombination between molecules containing attL and attR, in the presence of Int, IHF and Xis, can result in integrative recombination and the generation attP and attB. Hence, by flanking DNA segments with appropriate combinations of engineered att sites, in the presence of the appropriate recombination proteins, one can direct excisive or integrative recombination, as reverse reactions of each other.

Each of the att sites contains a 15 bp core sequence; individual sequence elements of functional significance lie within, outside, and across the boundaries of this common core (Landy (1989) *Ann. Rev. Biochem.* 58: 913). Efficient recombination between the various att sites requires that the sequence of the central common region be identical between the recombining partners, however, the exact sequence is now found to be modifiable. Consequently, derivatives of the att site with changes within the core are now discovered to recombine as least as efficiently as the native core sequences.

Integrase acts to recombine the attP site on bacteriophage lambda (about 240 bp) with the attB site on the E. *coli* genome (about 25 bp) (Weisberg and Landy (1983) In *Lambda II,* p. 211, Cold Spring Harbor Laboratory)), to produce the integrated lambda genome flanked by attL (about 100 bp) and attR (about 160 bp) sites. In the absence of Xis (see below), this reaction is essentially irreversible. The integration reaction mediated by integrase and

IHF works *in vitro,* with simple buffer containing spermidine. Integrase can be obtained as described by Nash (1983) *Meth. Enzym.,* 100: 210-216. IHF can be obtained as described by Filutowicz *et al.* (1994) *Gene* 147: 149-150.

In the presence of the .lambda. protein Xis (excise) integrase catalyzes the reaction of attR and attL to form attP and attB, *i.e.*, it promotes the reverse of the reaction described above. This reaction can also be applied in the present invention.

Numerous recombination systems from various organisms can also be used, based on the teaching and guidance provided herein *(see e.g.,* Hoess *et al.* (1986) *Nucleic Acids Res.* 14(6): 2287; Abremski *et al.* (1986) *J. Biol. Chem.* 261(1):391; Campbell (1992) *Bacteriol.* 174(23): 7495; Qian *et al.* (1992) *J. Biol. Chem.* 267(11): 7794; Araki *et al.* (1992) *J. Mol. Biol.* 225(1): 25). Many of these belong to the integrase family of recombinases (Argos *et al.* (1986) *EMBO J.* 5: 433-440). As indicated above, perhaps the best studied of these are the Integrase/att system from bacteriophage .lambda. (Landy (1993) *Current Opinions in Genetics and Devel.* 3: 699-707), the Cre/loxP system from bacteriophage P1 (Hoess and Abremski (1990) *In Nucleic Acids and Molecular Biology, vol. 4. Eds.:* Eckstein and Lilley, Berlin-Heidelberg: Springer-Verlag; pp. 90-109), and the FLP/FRT system from the *Saccharomyces cerevisiae* 2 mu circle plasmid (Broach et al. (1982) *Cell* 29: 227-234). Members of a second family of site-specific recombinases, the resolvase family (*e.g.*, gamma delta, Tn3 resolvase, Hin, Gin, and Cin) are also known and suitable for use in this invention. Although members of this highly related family of recombinases are typically constrained to intramolecular reactions (*e.g.*, inversions and excisions) and can require host-encoded factors, mutants have been isolated that relieve some of the requirements for host factors (Maeser and Kahnmann (1991) *Mol. Gen. Genet.* 230: 170-176), as well as some of the constraints of intramolecular recombination.

Other site-specific recombinases similar to lambda Int and similar to P1Cre can be substituted for Int and Cre. Such recombinases are known. In many cases the purification of such other recombinases has been described in the art. In cases when they are not known, cell extracts can be used or the enzymes can be partially purified using procedures described for Cre and Int.

While Cre and Int are described in detail for reasons of example, many related recombinase systems exist and their application to the described invention is also provided according to the present invention. As indicated above, the integrase family of site-specific recombinases can be used to provide alternative recombination proteins and recombination sites for the present invention, as site-specific recombination proteins encoded by bacteriophage lambda, phi 80, P22, P2, 186, P4 and P1. While group of proteins exhibits an unexpectedly large diversity of sequences all of these recombinases can be aligned in their C-terminal halves and this provides a means of identifying new recombinases.

A 40-residue region near the C terminus is particularly well conserved in all the proteins and is homologous to a region near the C terminus of the yeast 2 mu plasmid Flp protein. Three positions are perfectly conserved within this family: histidine, arginine and tyrosine are found at respective alignment positions 396, 399 and 433 within the well-conserved C-terminal region. These residues contribute to the active site of this family of recombinases, and suggest that tyrosine-433 forms a transient covalent linkage to DNA during strand cleavage and rejoining *(see, e.g., ,* Argos *et al.*(1986) *EMBO J.* 5:433-40).

Alternatively, IS231 and other *Bacillus thuringiensis* transposable elements could be used as recombination proteins and recombination sites. *Bacillus thuringiensis* is an entomopathogenic bacterium whose toxicity is due to the presence in the sporangia of delta-endotoxin crystals active against agricultural pests and vectors of human and animal diseases. Most of the genes coding for these toxin proteins are plasmid-borne and are generally structurally associated with insertion sequences (IS231, IS232, IS240, ISBT1 and

ISBT2) and transposons (Tn4430 and Tn5401). Several of these mobile elements have been shown to be active and participate in the crystal gene mobility, thereby contributing to the variation of bacterial toxicity.

Structural analysis of the iso-IS231 elements indicates that they are related to IS 1151 from *Clostridium perfringens* and distantly related to IS4 and IS186 from *Escherichia coli.* Like the other IS4 family members, they contain a conserved transposase-integrase motif found in other IS families and retroviruses.

Moreover, functional data gathered from IS231A in *Escherichia coli* indicate a non-replicative mode of transposition, with a preference for- specific targets. Similar results were also obtained in *Bacillus subtilis* and *B. thuringiensis* (see, e.g., Mahillon *et al.,* (1994) *Genetica* 93: 13-26; Campbell (1992) *J. Bacteriol.* 7495-7499.

In a preferred embodiment, the invention uses Cre/Lox driven recombination. Preferably the IoxP and IoxP 511 sites are employed as Lox sites. Various mutations of this sequence such as have been described in the literature *(see, e.g.,* Mack *et al., supra;* Hoess *et al.* (1986), *supra;* Hoess *et al.* (1984) *Biochem"* 81: 1026-29; Hoess *et al.* (1985) *Gene,* 40: 325-329; Abremski *et al.* (1986) *J*. *Biolog. Chem.,* 261: 391-396) are also suitable. Similar mutated sequences of IoxP, which are yet to be isolated also can be employed, so long as such sequences are capable of serving as recombining sites for Cre.

Intracellularly expressed recombinase is typically present in sufficient concentration to adequately drive recombination in the methods of this invention. Where an exogenous recombinase protein is supplied, the amount of recombinase that drives the recombination reaction can be determined by using known assays. Specifically, a titration assay can be used to determine the appropriate amount of a purified recombinase enzyme, or the appropriate amount of an extract.

### B) Engineering/modifying recombination sites.

The above recombinases and corresponding recombinase sites are suitable for use in generating diverse libraries according to the methods of this invention. However, wild-type recombination sites often contain sequences that reduce the efficiency or specificity of recombination reactions. For example, multiple stop codons in attB, attR, attP, attL and loxP recombination sites may occur in multiple reading frames on both strands, so recombination efficiencies are reduced, *e.g*., where the coding sequence must cross the recombination sites, (only one reading frame is available on each strand of loxP and attB sites) or impossible (in attP, attR or attL).

Accordingly, the present invention also provides engineered recombination sites that overcome these problems. For example, att sites can be engineered to have one or multiple mutations to enhance specificity or efficiency of the recombination reaction and the properties of product DNAs (*e.g.*, att1, att2, and att3 sites); to decrease reverse reaction (*e.g.*, removing Pland H1 from attB). The testing of these mutants determines which mutants yield sufficient recombinational activity to be suitable for recombination according to the present invention.

Mutations can be introduced into recombination sites for enhancing site specific recombination. Such mutations include, but are not limited to: recombination sites without translation stop codons that allow fusion proteins to be encoded; recombination sites recognized by the same proteins but differing in base sequence such that they react largely or exclusively with their homologous partners allow multiple reactions to be contemplated. Which particular reactions take place can be specified by which particular partners are present in the reaction mixture.

There are well known procedures for introducing specific mutations into nucleic acid sequences. A number of these are described in Ausubel *et al.* (1989-1996) *Current Protocols in Molecular Biology,* Wiley Interscience, New York. Mutations can be designed into oligonucleotides, which can be used to modify existing cloned sequences, or in amplification reactions. Random mutagenesis can also be employed if appropriate selection methods are available to isolate the desired mutant DNA or RNA. The presence of the desired mutations can be confirmed by sequencing the nucleic acid by well known methods.

A number of methods can be used to engineer a core region of a given recombination site to provide mutated sites suitable for use in the present invention. These include, but are not limited to mutation of the desired core sequence (*e.g.* via site-specific mutagenesis, error prone PCR, chemical mutagenesis, *etc)* or by recombination of two parental DNA sequences by site-specific *(e.g.* attL and attR to give attB) or other *(e.g.* homologous) recombination mechanisms.

The functionality of the mutant recombination sites can be demonstrated in ways that depend on the particular characteristic that is desired. For example, the lack of translation stop codons in a recombination site can be demonstrated by expressing the appropriate fusion proteins. Specificity of recombination between homologous partners can be demonstrated by introducing the appropriate molecules into in vitro reactions, and assaying for recombination products as described herein or known in the art. Other desired mutations in recombination sites might include the presence or absence of restriction sites, translation or transcription start signals, protein binding sites, and other known functionalities of nucleic acid base sequences. Genetic selection schemes for particular functional attributes in the recombination sites can be used according to known method steps. For example, the modification of sites to provide (from a pair of sites that do not interact) partners that do interact could be achieved by requiring deletion, via recombination between the sites, of a DNA sequence encoding a toxic substance. Similarly, selection for sites that remove translation stop sequences, the presence or absence of protein binding sites, *etc.,* can be easily devised by those skilled in the art.

### V Selection of molecules to be recombined (shuffled).

Virtually any nucleic acid or any molecule encoded by a nucleic acid can be be recombined ("shuffled") and selected according to the methods of this invention. It is noted that the nucleic acids include deoxyribonucleic acids, ribonucleic acids and, in some instances, peptide nucleic acids. The nucleic acids can include, but are not limited to genomic DNAs (gDNAs), CDNAs, mRNAs, reverse transcribed cDNAs, amplification products, and the like. Similarly, the polypeptides include natural and non-natural polypeptides.

Molecules subjected to recombination and/or subsequent selection according to the methods of this invention include, but are not limited to polypeptide ligands (*e.g.* normal and/or modified interleukins, growth factors, and the like), enzymes, receptor proteins, cell surface markers and/or antigens, antibodies and/or antibody fragments, nucleic acids encoding catalytic RNAs (*e.g.* ribozymes), catalytic DNAs, DNA binding sites (*e.g.* transcription factor and/or recombinase binding sites), and other regulatory elements (*e.g.*, promoters, enhancers, signals *etc*.). Single "functional" elements may be recombined in a single experiment, or related groups of elements (*e.g.* sets of promoters and enhancers in a metabolic pathway (*e.g.* a polyketide synthase)) may be recombined in a single recombination step.

In a particularly preferred embodiment, the methods of this invention are used to recombine antibody fragments to produce antibodies having particular binding specificities and/or avidities. The methods of this invention can be used to recombine VH and VL regions or fragments thereof. In preferred embodiments, recombination is between two or more elements including, but not limited to V_{H}, V_{L}, V_{H}, CDR₁, V_{H} CDR2, V_{H} CDR3, V_{L} CDR1, V_{L} CDR2, V_{L} CDR3, individual chains of a Fab fragment (*e.g.* V_{H}+C_{H}l and V_{L}+C_{L}), V_{H} dimers, V_{L} dimers, and the like. Again, as indicated above, multiple recombination events can occur at the same time. Thus, for example, V_{H} CDR1 and V_{L} CDR3 can both be recombined in the same experiment.

Antibody libraries for use in the methods of this invention can be obtained by methods well known to those of skill in the art *(see, e.g.,* Marks *et al.* (1991) *J. Mol. Biol.* 222*:* 581-597). In one embodiment, such methods involve isolating natural V_{H} and V_{L} repertoires present in cells (*e.g.* human peripheral blood lymphocytes) by PCR. The V-gene repertoires are spliced together at random using PCR to create a scFv gene repertoire which is cloned into vector (*e.g.* a phage vector) to create a large library of phage antibodies (*Id.*)*.*

Nucleic acids *(e.g.* encoding ligands, antigens, receptors, *etc.)* can be prepared using recombinant DNA techniques or chemically synthesized according to standard methods well known to those of skill in the art *(see, e.g.,* Sambrook, et al. (1989) *Molecular Cloning: a Laboratory Manual (2nd Ed., Vols.* 1-3), Cold Spring Harbor Laboratory, Berger & Kimel (1987) *Methods in Enzymology, Vol. 152: Guide to Molecular Cloning Techniques,* Academic Press, San Diego, Ausubel *et al.* (1987) *Current Protocols in Molecular Biology,* Greene Publishing and Wiley-Interscience, New York, *etc.* ).

The various molecules described above, and others, will be subjected to recombination and selection to provide new molecules having new and/or altered properties as described below.

### VI. Screening recombined polypeptides.

The proteins and/or nucleic acids recombined according to the methods of this invention can be screened for one or more properties. Those library members meeting the screening criteria can then be used, by themselves, for further founds of recombination and selection or can be used to enrich another library for further rounds of recombination and selection.

The library members can be screened for virtually any property or activity. Thus, for example, nucleic acid members can be screened for the ability to hybridise to particular target sequences under preselected hybridisation conditions, or they can be screened for catalytic (*e.g.* RNAase activity), or they can be screened for altered regulatory activity *(e.g.* response to particular promoters, particular tissue specificity, *etc.)* and the like. Similarly proteins can be screened for the ability to specifically bind to a particular target (*e.g.* target protein, receptor, glycoprotein, fat, *etc.)* at a selected minimum avidity, or for the ability to catalyse a particular chemical reaction under certain conditions (*e.g.* at a particular temperature, or pH, or in particular solvents (*e.g*. organic solvents, *etc.*) or under reducing or oxidizing conditions or in the presence of proteases, *etc.),* for stability under particular conditions (*e.g*. in the presence of certain denaturants, at particular pH, or extreme temperature, *etc*. ).

Thus, for example, in the case of antibodies or binding proteins, binding specificity and/or avidity can be determined in a BiaCore, a biosensor based on surface plasmon resonance. For this technique, the target (*e.g.* antigen, receptor, *etc*.) is coupled to a derivatized sensor chip capable of detecting changes in mass. When the library members are passed over the sensor chip, some library members binds to the immobilized target resulting in an increase in mass that is quantifiable. Measurement of the rate of association as a function of particular member concentration can be used to calculate the association rate constant (kₒₙ). After the association phase, buffer is passed over the chip and the rate of dissociation of antibody (k_{off}) determined. In certain embodiments, Kₒₙ is typically measured in the range 1.0 x 10² to 5.0 x 10⁶ and k_{off} in the range 1.0 x 10⁻¹ to 1.0 x 10⁻⁶. The equilibrium constant K_{d} is often calculated as k_{off}/kₒₙ and thus is typically measured in the range 10⁻⁵ to 10⁻¹². Affinities measured in this manner correlate well with affinities measured in solution by fluorescence quench titration.

Catalytic or enzyme activities can be measured by providing a appropriate substrate and buffer system and determining the rate of reaction (*e.g.* by monitoring the rate of loss of starting material or the rate of production of reaction product). Thus, for example, the activity of a protease recombined according to the methods of this invention can be determined by providing an appropriate polypeptide substrate bearing fluorescent molecules that quench each other. Then the library member(s) cleave the substrate, the fluorescent molecules are separated, cease quenching each other and a fluorescent signal is detectable *(see, e.g.* U.S. Patent 5,714,342). Similar measurements can be made for RNA or DNA catalytic activity.

Activity of elements controlling gene expression can be assayed using reporter genes *(i.e.* genes or cDNAs encoding a detectable product, *e.g.* β-galactosidase, green fluorescent protein, *etc*.) operably linked to the elements. Monitoring expression level of the reporter gene(s) under particular circumstances (*e.g.* in a particular tissue or cell, in the presence of a particular inducer, *etc*.) provides a measure of the activity of the control elements.

It is noted that this list of possible screening criteria and assays is illustrative and not intended to be limiting. The particular appropriate assay will be determined by the nature of the library member and the activity or property it is desired to evaluate. Assays for essentially any enzymatic or catalytic activity, physical or chemical property, or binding specificity and/or avidity are known to those of skill in the art and can be performed with at most routine experimentation.

While screening of the libraries of this invention can be performed manually it will be appreciated that many of such assays can be highly automated. Indeed, it is common to screen large combinatorial libraries now using "high throughput" techniques that allow several hundred thousand assays to be performed in as little as a week. High throughput assays for the presence, absence, or quantification of particular nucleic acids or protein products are well known to those of skill in the art. Similarly, binding assays and assays for particular chemical activities are similarly well known. Thus, for example, U.S. Patent 5,559,410 discloses high throughput screening methods for proteins and U.S. Patent 5,585,639 discloses high throughput screening methods for nucleic acid binding (*i.e.*, using high-density arrays), while U.S. Patents 5,576,220 and 5,541,061 disclose high throughput methods of screening for ligand/antibody binding.

High throughput screening systems can be custom designed for the particular assays of interest. Alternatively a number of general high-throughput systems can be adapted for a wide variety of assays. High throughput screening systems are commercially available (see, *e.g*., Zymark Corp., Hopkinton, MA; Air Technical Industries, Mentor, OH; Beckman Instruments, Inc. Fullerton, CA; Precision Systems, Inc., Natick, MA, etc.). These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. The manufacturers of such systems provide detailed protocols the various high throughput. Thus, for example, Zymark Corp. provides technical bulletins describing screening systems for detecting the modulation of gene transcription, ligand binding, and the like.

### VII. Kits.

In one embodiments, this invention provides kits for use in the methods of this invention. A preferred kit will typically comprise a container contain one or more libraries according to this invention. The libraries can take a number of forms. Thus, in one embodiment the library is a collection of cells containing members of a phage display library, while in another embodiment, the library consists of a collection of isolated phage. Still another library consists of a library of unpackaged nucleic acids (*e.g.* a plasmid library). The nucleic acids can be phagemid vectors encoding the desired recombination substrate(s) and ready for subcloning into a phage vector or the nucleic acids can be a collection of phagemid already carrying the subcloned nucleic acids.

The library can be a primary library comprising a population of individual nucleic acid molecules that has not yet been subjected to recombination as described herein, or alternatively, a secondary library comprising nucleic acid molecules derived from a primary gene library in which the diversity has been increased by the process of either site specific or general recombination.

In another embodiment, the kit may comprise a container containing vectors (*e.g.* plasmid, phagemid, *etc.)* containing appropriately located recombinase recognition sites and, optionally, providing polylinkers or other sites to facilitate cloning of one or more recombination substrates.

In addition the kits can optionally comprise one or more reagents (*e.g.* microtiter plates, buffers, cells, reporter molecules, antibiotics, *etc*.) suitable for practicing the methods described herein.

In addition, the kits may include instructional materials containing directions (*i.e.*, protocols) for the practice of the methods of this invention. Preferred instructional materials provide protocols for generating large and diverse nucleic acid libraries and/or for screening the members of such libraries. While the instructional materials typically comprise written or printed materials they are not limited to such. Any medium capable of storing such instructions and communicating them to an end user is contemplated by this invention. Such media include, but are not limited to electronic storage media (*e.g.,* magnetic discs, tapes, cartridges, chips), optical media (*e.g.*, CD ROM), and the like. Such media may include addresses to internet sites that provide such instructional materials.

### EXAMPLES

The following examples-are offered to illustrate, but not to limit the claimed invention.

### Example 1: Bacteria can be infected by more than a phagemid .

As a first step towards the development of an *in vivo* recombination system, the number of different phagemids which could infect single bacteria was assessed using five different phagemids expressing different antibiotic resistances: -ampicillin resistance (Bluescript: Stratagene, La Jolla), kanamycin resistance (pMPM-K1), tetracycline resistance (pMPM-T1), chloramphenicol resistance (pBSL121) and gentamycin resistance (pBSL141). pMPM plasmids are from Mayer (1995) *Gene* 163: 41-46 and the pBSL plasmids from Alexeyev *et al.* (1995) *Gene* 160: 63-67). By infecting bacteria with equal titres of these phagemids and plating on single or double antibiotics, and considering the number of colonies present on plates without antibiotics as 100%, it can be seen (Table 1), that most bacteria infected with two different phagemids have two different resistances.

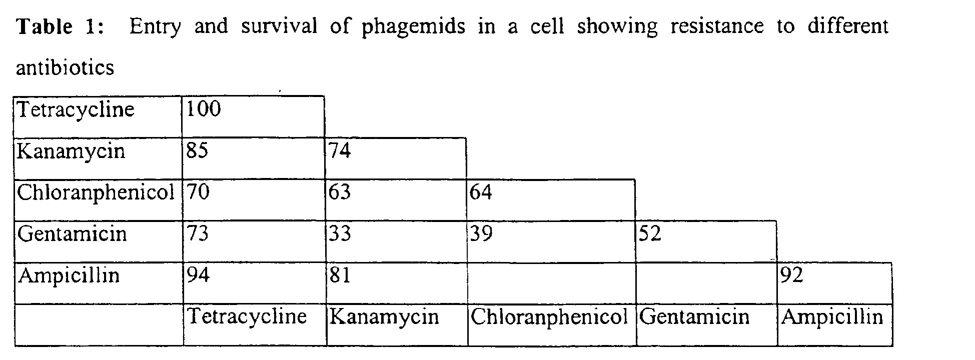

For example, 94% of all bacteria are resistant to ampicillin and tetracycline, and 85% are resistant to tetracycline and kanamycin. As a minimum estimate 10-40 % of bacteria appear to have had the potential to show resistance to all five antibiotics. Resistance to gentamycin with other antibiotics was found most infrequently, but this is probably a characteristic of resistance to this antibiotic, as only 52% of bacteria could be rendered gentamycin resistant, even when used alone. Resistance to some antibiotic pairs is not indicated as the plasmids used did not permit us to test these combinations.

These data show that infection using phagemids is an efficient method to introduce at least five different nucleic acid molecules into a cell.

### Example 2: Intracellular recombination can be used to build a functional antibody with its chain components present on separate phagemids

In order to use cre recombinase to shuffle antibody variable region genes in the scFv format, a lox site was placed between the heavy and light chain genes. This involved the use of the translated lox site as a protein linker. An examination of the six possible frames available for the wild type loxP site and the mutated loxP 511site (which will not recombine with the wild type loxP (Hoess and Abremski (1985) *J Mol Biol,* 181: 351-362) identified a translation of loxP 511 (ITSYNVYYTKL, SEQ ID NO:1) which had only a single basic amino acid (to reduce the possibility of proteolysis), lacked stop codons and was the least hydrophobic. The sequence of the linker as used in the scFvs is given in Figures 2 and 3. The ability of this sequence to act as a scFv linker as well as two commonly used linkers, (gly₄ser)₃ (SEQ ID NO:2) (Bird *et al.* (1988) [published erratum appears in *Science* 1989 Apr. 28;244(4903):409]. *Science* 242:423-426) and 220, a modified version of the 218 linker (Whitlow (1993) *Protein Engineering,* 6: 989-995), was carried out by creating small scFv libraries for each of these linkers and assessing display levels by western blots developing with the SV5 antibody which recognizes the tag between the cloned scFv and gene 3 protein. The loxP 511 linker showed display levels as good as, or better, than the two other more widely used linkers.

On the basis of this result, a new phagemid display vector, pDAN5, was designed and constructed (Figures 3, 4, SEQ ID NO:3). (Note the sequence illustrated is the pDAN5 D1.3 vector which has the D1.3 scFv cloned in. Nucleotides 102-426 are the D1.3 VL gene and nucleotides 490-837 are the D1.3 VH gene. The basic pDAN5 vector (absent antibody) doe s not include nucleotides 102-426 and 490-837, and will include polylinker sequences in their place). This vector was tested for the ability to display three different scFvs derived from previously characterized monoclonal antibodies. The results show that all three scFvs recognize the appropriate antigens when displayed in this new vector using the loxP 511 linker. ELISA signals obtained using this vector were similar to those obtained using display vectors with the gly-ser linker.

To make pDAN5, a new polylinker was cloned into pUC119 using EcoRI and HindIII by overlap PCR of two long oligonucleotides. This introduced the bacterial leader sequence, a polycloning cassette containing the restriction sites indicated in bold in Figure 3, the SV5 tag (Hanke *et al.* (1992) *J Gen virol,* 73: 653-660.), a His₆ (SEQ ID NO:4) tag and an amber stop codon (see Figures 3 and 4). Gene 3 was subsequently cloned into this polylinker by PCR amplification from fdtet using NotI and EcoRI. The 5' end of mature gene 3 was inserted downstream of the amber stop codon, and a wild type lox sequence was inserted at the 3' end of gene 3 after the stop codon and before the EcoRI site by PCR. The D1.3. scFv was assembled (in the order VL-VH) from D1.3 scFv (VH-VL order) amplifying VH D1.3 with VHback-DAN and VHfor-2-DAN, and VK D1.3 with VK2back-DAN and VK2for-DAN (Table 2).

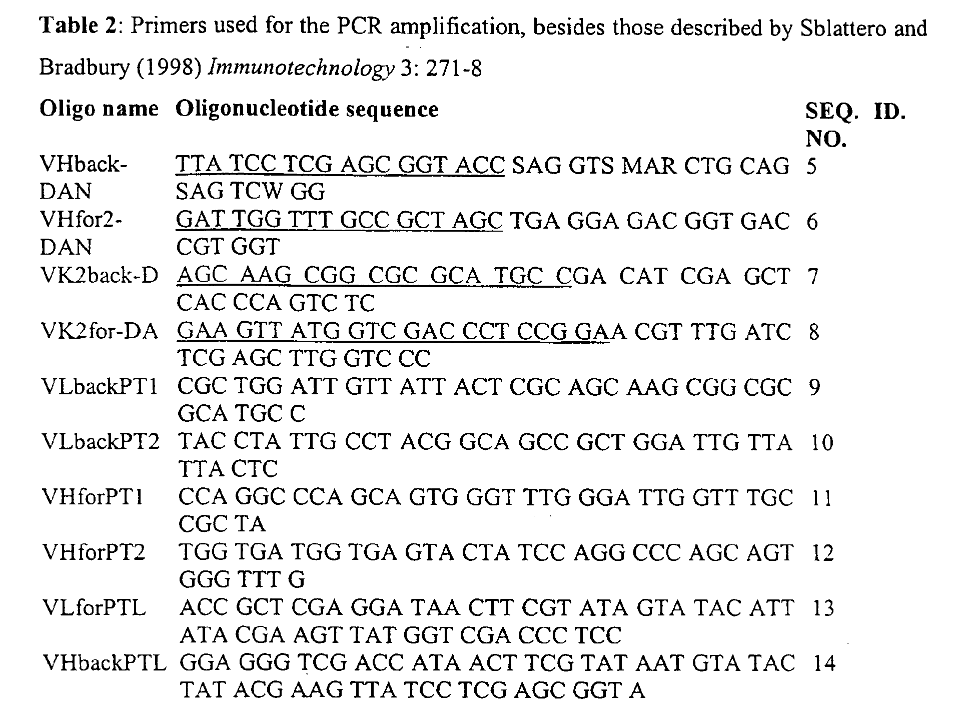

All V genes were gel purified and approximately 200ng were used as templates for further amplification to add a region of overlap in the scFv linker as well as long tails to facilitate restriction enzyme digestion. The primers VLbackPT1 and VLforPTL were used to amplify VL genes and VHforPT1 and VHbackPTL for the VH genes. Amplified bands were gel purified as below.

The D1.3 scFv was assembled by mixing equal amounts (50-200ng) of VH and VL genes and performing assembly essentially as described in (Krebber *et al.* (1997) *J. Immunol. Meth.* 201: 35-55): 8 cycles of PCR without primers followed by 25 cycles in the presence of VLbackPT2 and VHforPT2. Cycling parameters were 94°C for 1 min (denaturation), 60°C for 1 min (annealing) and 72°C for 1' 30" (extension).

The amplified scFv was digested with BssHII and NheI and ligated into BssHII / NheI cut pDAN5. The ligation mix was electroporated into electrocompetent DH5αF and plated on 2XTY 100µg/ml ampicillin / 1% glucose plates, clones were confirmed by sequencing.

The ability to shuffle heavy and light chain V genes in vivo to create functional antibodies was tested using the scFv derived from the anti-lysosyme mAb, D1.3. Two scFvs which contained either D1.3 VH or D1.3 VL with irrelevant partner chains were created (VL/D1.3-VH/X and VL/Y-VH/D1.3) by PCR cloning. Recognition of lysosyme by D1.3 scFv was shown to require the presence of both D1.3 heavy and light chains; single D1.3 chains associated with irrelevant partner chains were non-functional. An outline of the scheme used is indicated in Figure 5.

10ml E. coli BS1365, which expresses cre recombinase constitutively (BS1365: BS591 F' kan (BS591: recAl endA1 gyrA96 thi-1 D IacU169 supE44 hsdR17 [lamdalmm434 nin5 X1-cre] (Sauer and Henderson (1988) *Gene* 70: 331-341)) was grown to OD550 0.5 at 37°C in 2XTY 100µg/ml kanamycin /1% glucose.

Equal amounts of phagemid containing the two scFv genes (VL/D1.3-VH/X and VL/Y-VH/D1.3) were added to the bacteria at an MOI of 20:1 (5x10¹⁰ of each phagemid added to 5x10⁹ bacteria). This was left for 30 minutes at 37°C without shaking to allow infection to occur.

Ampicillin was added to 100µg/ml and bacteria were grown for at least 12 hours at 30°C. Recombination occurs during this period.

After growth for 12 hours, bacteria were diluted 1:20 in 10ml of the same growth medium, grown to OD550 0.5 and M13 K07 helper phage added at MOI of 20:1.

This was left for 30 minutes at 37°C without shaking to allow infection to occur.

The culture was grown for 6-18 hours at 30°C, centrifuged at 4000 rpm for 15 mins and the supernatant taken. This step prepares phagemid which do not display scFv, with phenotype or genotype (usually not) coupled.

10ml DH5αF was grown to OD550 0.5 in 2XTY at 37°C.

Phagemid prepared in step 6 was added to the DH5αF at MOI less than 1, left for 30 minutes at 37°C and plated on 2XTY 100µg/ml ampicillin / 1% glucose plates. This step couples phenotype to genotype, in the absence of this step, the displayed scFv may not necessarily correspond to the scFv gene within the phagemid.

Phagemid displaying scFv were made from 20 individual colonies by growing to OD550 0.5 in 10ml 2XTY 100µg/ml ampicillin /1% glucose, infecting with M13K07 at MOI 20:1 for 30 minutes at 37°C, centrifuging and resuspending the bacteria in 10ml 2XTY 100µg/ml ampicillin. The cultures were grown for 6-18 hours at 30°C.

If recombination was successful, each bacteria should contain four different scFv genes (V_{L}/D1.3-V_{H}/D1.3; V_{L}/D1.3-V_{H}/X; V_{L}/Y-V_{H}/D1.3 and V_{L}/Y-V_{H}/X). (see Figure 5). In the presence of cre recombinase, 25% (16/64) of the bacterial colonies obtained in step 8/9 were shown to have undergone recombination by PCR. Furthermore, it was shown that 25% of phagemid prepared in step 9 could recognize lysozyme by ELISA. In bacteria not expressing cre, no recombination was found and no functional D1.3 identified, by either PCR or ELISA. This shows that recombination was induced as predicted in those cells expressing cre, at the loxP sites found between V_{L} and V_{H} and at the end of gene 3, the net result being an exchange of V_{H} (and attached gene 3, which is constant) between different phagemids.

### Example 3: Creation of a large gene library of antibodies in pDAN5 by means of intracellular recombination.

### A) Creation of a primary scFv gene library by standard cloning techniques.

A primary scFv phagemid gene library consisting of 7 x 10⁷ independent clones, was created in pDAN5 by cloning PCR assembled scFv derived from peripheral blood Vµ, Vλ, and Vκ (Vmu, Vlambda, and Vkappa) genes in the following way:
1 Human peripheral blood lymphocytes were prepared by density gradient centrifugation on Ficoll Hypaque (Pharmacia).
2 Total RNA was prepared from these lymphocytes by acid guanidinium thiocyanate, phenol chloroform extraction and isopropanol precipitation (Chomczynski and Sacks (1987) *Anal. Biochem.* 162: 156-159).
3 cDNA was prepared using SuperScript II RNase H- Reverse Transcriptase (Gibco BRL) with random hexamers starting with 1-5µg of total RNA in a final volume of 20µl following instructions provided with the SuperScript.
4 IgM VH genes were first amplified from 0.5µl cDNA reaction, using IgMfor and the VHback primers described in (Sblattero and Bradbury (1998) *Immunotechnology* 3: 271-8). Reaction volumes were 20 µl, using 0.5 µl of cDNA reaction, 10 pmol of each primer, 200 µM dNTPs, 2µl 10X PCR buffer, and 0.5 µl (2.5 U) of Taq DNA polymerase (Perkin Elmer): Cycling parameters were 94°C for 1 min (denaturation), 55°C for 1 min (annealing) and 72°C for 1' (extension) for thirty cycles. All 20µl were loaded on a 2% agarose gel (FMC) and gel purified using the Qiagel purification kit (Qiagen). Subsequently, VH genes were reamplified using the VHfor mix of primers with the individual VHback primers described in (Sblattero and Bradbury, 1998] in 50µl volumes using 1 µl of purified VH (other parameters as before).
5 Vlambda and Vkappa genes were similarly amplified (using individual VLback primers with the mix of VL for primers) from the previously derived cDNA.
6 All V genes were gel purified and approximately 200ng were used as templates for further amplification to add a region of overlap in the scFv linker as well as long tails to facilitate restriction enzyme digestion. The primers VLbackPT1 and VLforPTL were used to amplify VL genes and VHforPTI and VHbackPTL for the VH genes. Amplified bands were gel purified as above.
7 The scFv library was assembled by mixing equal amounts (200-500ng) of VH and VL genes and performing assembly essentially as described in (Krebber et al., 1997): 8 cycles of PCR without primers followed by 25 cycles in the presence of VLbackPT2 and VHforPT2. Cycling parameters were 94°C for 1 min (denaturation), 60°C for 1 min (annealing) and 72°C for 1' 30" (extension).
8 The amplified scFv were digested with BssHII and NheI and ligated into BssHII / NheI cut pDANS-D1.3. The ligation mix was electroporated into electrocompetent DH5αF and plated on 2XTY 100µg/ml ampicillin / 1% glucose plates to obtain a primary library consisting of 7x10⁷ independent clones.
9 The colonies were scraped up in 2XTY 10% glycerol and frozen down in 1ml aliquots.

### B) Intracellular recombination to create a large secondary gene library.

To create the large secondary library the scheme illustrated in Figure 6 was followed. The detailed protocol is as below:
1. 100µl of primary library bacteria were diluted in 100ml 2XTY 100µg/ml ampicillin /1% glucose and grown to an OD550 0.5 and M13 K07 helper phage added at MOI of 20:1.
2. This was left for 30 minutes at 37°C without shaking to allow infection to occur.
3. Kanamycin was added to 100µg/ml and the culture was grown for 5-18 hours at 30°C, centrifuged at 4000 rpm for 15 mins and the supernatant taken. This step prepares phagemid which do not display scFv, but do contain scFv genes.
4. 20 ml of E. coli BS1365, which expresses cre recombinase constitutively was grown to OD550 0.5 at 37°C in 2XTY 100µg/ml kanamycin / 1% glucose.
5. Phagemid prepared in step 3 were added at an MOI of 200:1 (2×10¹² phagemid were added to 1×10¹⁰ bacteria). This was left for 30 minutes at 37°C without shaking to allow infection to occur. This resulted in infection of bacteria by more than one phagemid.
6. Ampicillin was added to 100µg/ml and bacteria were grown for at least 12 hours at 30°C. Recombination occurs during this period.
7. After growth for about 12 hours, bacteria were added to 380ml of the same growth medium (1/20 dilution), grown to OD550 0.5 at 37°C and M13 K07 helper phage added at MOI of 20:1. Bacteria were grown for a further 6-18 hours, and phagemid prepared as described above. This produces a genetically diverse recombined antibody phagemid library, but with no displayed protein (which would anyway have phenotype and genotype uncoupled). This is overcome by reinfecting DH5αF at an MOI less than 1.
8. 1 litre of DH5αF' were grown to OD550 0.5 in 2XTY / 1% glucose at 37°C, 5x10¹¹ phagemid were added (MOI less than 1) and left to stand for 30 minutes to allow infection to occur. M13K07 were added at an MOI of 20:1, the culture was left to stand for 30 minutes at 37°C to allow infection to occur, and then centrifuged 4000rpm for 15 minutes. Bacteria were resuspended in 1 litre 2XTY 100µg/ml ampicillin / 100µg/ml kanamycin and grown for 6-18 hours at 30°C. The culture was centrifuged (4000rpm 30 minutes) and phagemid harvested from the supernatant.
9. 150ml 2.5M NaCl 40%PEG 8000 were added to the supernatant (this precipitates phagemid). The pellet was rescued by centrifugation (4000rpm 15minutes), resuspended in 50ml PBS and centrifuged (9000rpm 15 minutes) to remove bacteria and aggregated phagemid.
10. Step 9 was repeated by adding 10ml 2.5M NaCl 40%PEG 8000 to the 50ml phagemid preparation. The final PEG precipitate was resuspended in 20ml and phagemid further purified by cesium chloride density centrifugation as described in (Smith, and Scott (1993) *Meth. Enzymol.,* 217: 228-157) and resuspended in 20ml. This constitutes the final antibody phagemid library which was used for selections.

### C) Assessment of diversity in individual cells

The experiments described in examples 1 and 2 above, indicated that at least five different phagemid could enter a single bacteria, and that two phagemid that entered a single bacteria were able to recombine with one another to equilibrium, resulting in four different phagemid. However, they did not give an indication of how long such phagemid would survive after growth in either liquid or solid culture. In order to clarify this point, and characterise the potential diversity of the gene library produced, phagemid were produced from a single colony of cre bacteria containing phagemids which have undergone recombination. These phagemid were used to obtain single bacterial colonies each containing only one type of phagemid. Analysis of these different colonies by PCR allowed the identification of the different VH and VL chains present in the original cre colony (see Figure 6 for details).

After growth for at least 12 hours in cre expressing bacteria, the culture was plated out to isolate individual colonies. If recombination has been successful, and all phagemid are still present, each of these colonies should contain many phagemid with multiple recombined V genes. In order to identify these, phagemid were first prepared from a single colony (these should consist of all the different scFv combinations which have arisen within the original starting cell), and then these were used to prepare colonies containing single phagemids. In this way it is possible to isolate the many single scFv genes found in the starting colony.

Briefly this was performed as follows:
1. After infection at high MOI, and growth overnight, cre expressing bacteria were plated out on 2XTY 100µg/ml kanamycin / 100µg/ml ampicillin / 1% glucose plates to isolate individual colonies.
2. Phagemid were prepared from these individual colonies by growing such colonies to OD550 0.5 in 1ml 2XTY 100µg/ml kanamycin / 100µg/ml ampicillin / 1% glucose at 37°C, infecting with M13K07 and using the techniques described above to produce phagemid.
3. To derive colonies from these phagemid, DH5αF bacteria were grown to an OD550 0.5 in 2XTY at 37°C and infected with the phagemid obtained in step 2 at an MOI less than 1. Such colonies contain single phagemids, and the complete population of colonies represent the diversity of the phagemids contained within the single starting bacteria grown up in step 2.
4. Individual V_{H} and V_{L} chains present in each phagemid were identified by PCR amplification and fingerprinting with BstNI or by sequencing.

Two cells were taken for analysis and the results for both are shown in Figure 7. Both cells gave very similar results, with 17-18 V_{H} genes and 12-14 V_{L} genes identified per cell.

These different V genes are present in 28-29 different combinations, with many of the different V genes found in more than one scfv combination. In the case of the first cell illustrated, all four combinations of two V_{H}/V_{L} pairs can be identified, indicating that recombination has been extensive. In both cells, over 50% of the V_{H}/V_{L} pairs identified are present in single copies, suggesting that the small sample (37-41 scFv) analyzed has not identified the full complement of V_{H} and V_{L} genes or their combinations, and that the true diversity is higher than that identified.

The degree of diversity created in a single cell was difficult to assess. It is very likely that the 18 different V_{H} genes rescued from a single colony were matched by 18 different V_{L} genes. The potential diversity identified in this small sample is 324 (18²). It is likely that not all the different V genes present were identified, and it is known that V genes can have identical fingerprints but different sequences, suggesting that the diversity created in a single cell may exceed this, giving a maximum estimate which approaches the 500-700 copy number of pUC based plasmids (Sambrook *et al.,* 1989 *supra.).* The lowest estimate is that which was observed on the basis of the fingerprint analysis: 28-29 different scFv combinations per cell.

The degree of recombination was extensive, with many different groups of three out of four combinations identified. One light chain was found with nine different heavy chains, and one heavy chain with five different light chains. The fact that so many different plasmids can stably co-exist in a single cell is somewhat surprising, and at odds with the dogma which states that only one plasmid per complementation group is able to survive in a bacterial cell. However, it is likely that once a large number of V genes have been introduced into a cell, ongoing recombination will both maintain the diversity as well as prevent selective pressure eliminating individual V genes. This gives a potential range of diversity created by a single cell of 29-500 different scFvs.

In the library created here, 10¹⁰ (20ml) bacteria expressing the cre recombinase were infected with 2x10¹² phage (an MOI of 200). On the basis of the diversity actually observed (each bacteria produces 29 different phage), the final library size will be 2.9x10¹¹ (29 x 10¹⁰). While if all 324 possible V gene combinations are present, the library will be approximately ten times higher (3.2x10¹²). The size of the library which can be used practically, however, is limited by the reinfection step at low MOI when phenotype and genotype are coupled. By using one litre to perform this step, it cannot exceed 5x 10¹¹, the number of bacteria in one litre, although by using larger volumes, larger libraries can be easily created.

### D) Testing the large phage antibody library

The library was tested by selection on a number of different antigens (*see* Table 3) and antibodies were isolated against all in two cycles. In each case at least 3 different scFvs were obtained per antigen with an average of 6 per antigen.

**Table 3. Antigens for which antibodies have been selected**

| Antigen | % positive after 2-3 turns of selection | numbers of independent scFv | Affinity (nM) |
|---|---|---|---|
| Human | | | |
| albumin serum | 96 | 7 | |
| Cyclin D | 50 | 4 | |
| Cdk2 | 96 | 9 | 29, 59, 82 |
| Rad52 | 80 | 11 | 14 |
| Endonuclease Flap | 50 | | |
| Ku70/80 | 25 | | |
| cdc25A | 25 | 3 | |
| cdc25C | 13 | | |
| PARP DNA binding domain | 38 | | |
| PARP 85kDa | 68 | | |

| Rhodococcus | | | |
|---|---|---|---|
| pigs10:ferredoxin pyruvate oxyreductase | 38 | | |
| pigs 12B:phosphoglycerol dehydrogenases | 50 | 4 | |

| Miscellaneous | | | |
|---|---|---|---|
| HIV1 loop | 75 | 6 | |

Selection was performed as follows:
1. An immunotube (Nunc) was coated with 1ml of the antigen of interest at 10µg/ml overnight at room temperature. After coating the tube was washed out with PBS three times and blocked by filling the immunotube with 2% Marvel / PBS (MPBS). Simultaneously, 1ml of library (containing 5x10¹² - 10¹³ phagemid) was blocked by adding 10% Marvel / PBS to a final concentration of 2%.
2. After blocking, the immunotube was washed 3 times with PBS and the library was added and incubated 1 hour standing and 1 hour rotating slowly end over end.
3. After selection, the tube was washed ten times with PBS and ten times with 0.1% Tween 20 / PBS.
4. Binding phagemid were eluted with 1ml 100mM triethanolamine for 8 minutes at room temperature, and immediately transferred to a new tube and neutralized with 1M Tris pH 7-7.5.
5. Eluted phagemid were added to DH5αF at OD550 0.5 and left without shaking for 30 minutes at 37°C.
6. The infected bacteria were plated on 2XTY / 100µg/ml ampicillin / 1% glucose plates and grown for 12-18 hours at 30°C.
7. Bacteria were scraped up in 10ml 2XTY, thoroughly mixed and diluted in 50ml of 2XTY / 100µg/ml ampicillin /1% glucose to an OD550 0.05, and phagemid were prepared by growing at 37°C to an OD550 0.5 infecting with M13K07 and following the protocol described above, including two precipitations with PEG/NaCl resulting in a final volume of 1 ml phagemid.
8. 10¹² of these phagemid were used as input for the next round of selection which was performed exactly as described above, except that in step 3, washing was performed twenty times with PBS and ten times with 0.1% Tween 20 / PBS followed by a long wash of 30-60 minutes in PBS at room temperature.
9. Eluted phage were plated out at low density in order to isolate individual colonies, phagemid were made from these and tested in ELISA as described in (Marks et al., 1991).

### Example 4: In vivo random recombination without specific recombinases

Wild type beta lactamase is unable to give resistance to the antibiotic cefotaxime except at a concentration of only 0.025 µg/ml, which is no greater than that provided by bacteria not harboring this resistance gene. It has been shown that resistance to cefotaxime can be produced by mutation of the beta lactamase gene in a number of key residues, this being done by in vitro recombination, but not by error prone PCR alone (Stemmer 1994).

In order to demonstrate *in vivo* random recombination without specific recombinases (*i.e*. using the endogenous cellular recombination machinery), the gene for beta lactamase (encoding ampicillin resistance) was amplified from the vector pBSL167 (Alexeyev *et al.* (1995)) *Gene* 160: 63-67) using error prone PCR conditions. After transfection a primary library of 3x 10⁵ independent clones was obtained. Phagemid were obtained from these and used to infect bacteria at different phagemid:bacteria ratios. These bacteria were grown for at least 12 hours and phagemid prepared from them. It was found that those phagemid prepared from bacteria previously infected at high phagemid:bacteria ratios (greater than 10:1) were able to give rise to many bacterial colonies with resistance to 0.25µg/ml cefotaxime, whereas those phagemid prepared from bacteria previously infected at low phagemid:bacteria ratios (less than 1:1) were able to give far fewer colonies resistant to 0.25µg/ml cefotaxime. Recombination is only able to occur where more than one phagemid is present within a single bacterium, and useful recombination is only able to occur if at least two phagemid with complementary mutations (mutations which if found on the same beta lactamase gene, rather than two different ones, are able to give rise to a greater resistance to cefotaxime) are present in the same bacterium. As the only difference between those phagemids able to give rise to more resistant colonies and those able to give rise to few resistant colonies is the multiplicity of infection, it is clear that recombination has occurred in the former case, but not the latter.

The protocol followed is as follows:
1. The beta lactamse gene was amplified under error prone PCR conditions with two primers hybridizing to the polylinker in the plasmid pBSL167.
2. The amplified band was cut with XsacI and KpnI and recloned back into pBSL167 cut with the same enzymes to give rise to a primary library of 3x10⁵ colonies on chloramphenicol plates (34µg/ml). Resistance to chloramphenicol is provided by the chloramphenicol acetyl transferase gene found in the backbone of the vector.
3. The bacteria were scraped up in 2XTY, diluted to OD550 0.1 in 10ml 2XTY 50µg/ml chloramphenicol, grown to OD550 0.5 at 37°C and 10¹¹ helper phage added. After 30 minutes incubation at 37°C, kanamycin was added to 25µg/ml.
4. Bacteria were grown for at least 12 hours at 37°C, and phagemid prepared from the culture. The culture was centrifuged (3500rpm 20 minutes), the supernatant treated with 2.5ml 2.5M NaCl 40%PEG 8000 (this precipitates phagemid). The pellet was rescued by centrifugation (3000rpm 20 minutes), resuspended in 1ml PBS and centrifuged (13000rpm 10 minutes) to remove bacteria and aggregated phagemid. This constitutes the primary phagemid library.
5. A number of aliquots of 10ml DH5αF' bacteria were grown to an OD550 0.5 in 2XTY at 37°C. Aliquots of the primary phagemid library were added to these bacteria at different phagemid: bacteria ratios, ranging from 0.1:1 to 100:1. The bacteria were left for 30 minutes at 37°C to allow infection to occur, chloramphenicol was added and the bacteria grown at 37°C for at least 12 hours.
6. After growth, phagemid were prepared from the bacterial supernatant as described in steps 3-4 above. These constitute the secondary or recombined library.
7. In order to test the ability of the secondary or recombined library to confer a greater resistance to cefotaxime, Dh5αF' bacteria were grown to OD550 0.5 in 2XTY at 37°C, and phagemid from the different secondary libraries (produced at different phagemid:bacteria ratios) were added to 1ml of DH5αF' (*i.e.*, at a phagemid:bacteria ratio of less than 1 to ensure that each bacteria is infected by, on average, only one phagemid). The culture was left for 30 minutes at 37°C, and plated on cefotaxime plates of different concentrations.
8. Secondary libraries created from bacteria infected at phagemid:bacteria ratios of greater than 10:1 gave rise to 100-800 colonies on 0.25 µg/ml cefotaxime, whereas those libraries created from bacteria infected at phagemid:bacteria ratios of less than 1 did not give any resistant colonies.

It is understood that the examples and embodiments described herein are for illustrative purposes only and that various modifications or changes in light thereof will be suggested to persons skilled in the art.

### SEQUENCE LISTING

Sequence ID No: 1
   ITSYNVYYTKL
Sequence ID No: 2 (gly₄ser)₃
   GGGGS GGGGS GGGGS
Sequence ID No: 3. pDAN5 D1.3 
CTAGCGGCAA ACCAATCCCA AACCCACTGC TGGGCCTGGA TAGTACTCAC CATCACCATC ACCATTAGGC GGCCGCTACT
GTTGAAAGTT GTTTAGCAAA ACCTCATACA GAAAATTCAT TTACTAACGT CTGGAAAGAC GACAAAACTT TAGATCGTTA
CGCTAACTAT GAGGGCTGTC TGTGGAATGC TACAGGCGTT GTGGTTTGTA CTGGTGACGA AACTCAGTGT TACGGTACAT
GGGTTCCTAT TGGGCTTGCT ATCCCTGAAA ATGAGGGTGG TGGCTCTGAG GGTGGCGGTT CTGAGGGTGG CGGTTCTGAG
GGTGGCGGTA CTAAACCTCC TGAGTACGGT GATACACCTA TTCCGGGCTA TACTTATATC AACCCTCTCG ACGGCACTTA
TCCGCCTGGT ACTGAGCAAA ACCCCGCTAA TCCTAATCCT TCTCTTGAGG AGTCTCAGCC TCTTAATACT TTCATGTTTC
AGAATAATAG GTTCCGAAAT AGGCAGGGTG CATTAACTGT TTATACGGGC ACTGTTACTC AAGGCACTGA CCCCGTTAAA
ACTTATTACC AGTACACTCC TGTATCATCA AAAGCCATGT ATGACGCTTA CTGGAACGGT AAATTCAGAG ACTGCGCTTT
CCATTCTGGC TTTAATGAGG ATCCATTCGT TTGTGAATAT CAAGGCCAAT CGTCTGACCT GCCTCAACCT CCTGTCAATG
CTGGCGGCGG CTCTGGTGGT GGTTCTGGTG GCGGCTCTGA GGGTGGCGGC TCTGAGGGTG GCGGTTCTGA GGGTGGCGGC
TCTGAGGGTG GCGGTTCCGG TGGCGGCTCC GGTTCCGGTG ATTTTGATTA TGAAAAAATG GCAAACGCTA ATAAGGGGGC
TATGACCGAA AATGCCGATG AAAACGCGCT ACAGTCTGAC GCTAAAGGCA AACTTGATTC TGTCGCTACT GATTACGGTG
CTGCTATCGA TGGTTTCATT GGTGACGTTT CCGGCCTTGC TAATGGTAAT GGTGCTACTG GTGATTTTGC TGGCTCTAAT
TCCCAAATGG CTCAAGTCGG TGACGGTGAT AATTCACCTT TAATGAATAA TTTCCGTCAA TATTTACCTT CTTTGCCTCA
GTCGGTTGAA TGTCGCCCTT ATGTCTTTGG CGCTGGTAAA CCATATGAAT TTTCTATTGA TTGTGACAAA ATAAACTTAT
TCCGTGGTGT CTTTGCGTTT CTTTTATATG TTGCCACCTT TATGTATGTA TTTTCGACGT TTGCTAACAT ACTGCGTAAT
AAGGAGTCTT AAGCATGCAT AACTTCGTAT AATGTATGCT ATACGAAGTT ATGAATTCAC TGGCCGTCGT TTTACAACGT
CGTGACTGGG AAAACCCTGG CGTTACCCAA CTTAATCGCC TTGCAGCACA TCCCCCTTTC GCCAGCTGGC GTAATAGCGA
AGAGGCCCGC ACCGATCGCC CTTCCCAACA GTTGCGCAGC CTGAATGGCG AATGGCGCCT GATGCGGTAT TTTCTCCTTA
CGCATCTGTG CGGTATTTCA CACCGCATAC GTCAAAGCAA CCATAGTACG CGCCCTGTAG CGGCGCATTA AGCGCGGCGG
GTGTGGTGGT TACGCGCAGC GTGACCGCTA CACTTGCCAG CGCCCTAGCG CCCGCTCCTT TCGCTTTCTT CCCTTCCTTT
CTCGCCACGT TCGCCGGCTT TCCCCGTCAA GCTCTAAATC GGGGGCTCCC TTTAGGGTTC CGATTTAGTG CTTTACGGCA
CCTCGACCCC AAAAAACTTG ATTTGGGTGA TGGTTCACGT AGTGGGCCAT CGCCCTGATA GACGGTTTTT CGCCCTTTGA
CGTTGGAGTC CACGTTCTTT AATAGTGGAC TCTTGTTCCA AACTGGAACA ACACTCAACC CTATCTCGGG CTATTCTTTT
GATTTATAAG GGATTTTGCC GATTTCGGCC TATTGGTTAA AAAATGAGCT GATTTAACAA AAATTTAACG CGAATTTTAA
CAAAATATTA ACGTTTACAA TTTTATGGTG CACTCTCAGT ACAATCTGCT CTGATGCCGC ATAGTTAAGC CAGCCCCGAC
ACCCGCCAAC ACCCGCTGAC GCGCCCTGAC GGGCTTGTCT GCTCCCGGCA TCCGCTTACA GACAAGCTGT GACCGTCTCC
GGGAGCTGCA TGTGTCAGAG GTTTTCACCG TCATCACCGA AACGCGCGAG ACGAAAGGGC CTCGTGATAC GCCTATTTTT
ATAGGTTAAT GTCATGATAA TAATGGTTTC TTAGACGTCA GGTGGCACTT TTCGGGGAAA TGTGCGCGGA ACCCCTATTT
GTTTATTTTT CTAAATACAT TCAAATATGT ATCCGCTCAT GAGACAATAA CCCTGATAAA TGCTTCAATA ATATTGAAAA
AGGAAGAGTA TGAGTATTCA ACATTTCCGT GTCGCCCTTA TTCCCTTTTT TGCGGCATTT TGCCTTCCTG TTTTTGCTCA
CCCAGAAACG CTGGTGAAAG TAAAAGATGC TGAAGATCAG TTGGGTGCAC GAGTGGGTTA CATCGAACTG GATCTCAACA
GCGGTAAGAT CCTTGAGAGT TTTCGCCCCG AAGAACGTTT TCCAATGATG AGCACTTTTA AAGTTCTGCT ATGTGGCGCG
GTATTATCCC GTATTGACGC CGGGCAAGAG CAACTCGGTC GCCGCATACA CTATTCTCAG AATGACTTGG TTGAGTACTC
ACCAGTCACA GAAAAGCATC TTACGGATGG CATGACAGTA AGAGAATTAT GCAGTGCTGC CATAACCATG AGTGATAACA
CTGCGGCCAA CTTACTTCTG ACAACGATCG GAGGACCGAA GGAGCTAACC GCTTTTTTGC ACAACATGGG GGATCATGTA
ACTCGCCTTG ATCGTTGGGA ACCGGAGCTG AATGAAGCCA TACCAAACGA CGAGCGTGAC ACCACGATGC CTGTAGCAAT
GGCAACAACG TTGCGCAAAC TATTAACTGG CGAACTACTT ACTCTAGCTT CCCGGCAACA ATTAATAGAC TGGATGGAGG
CGGATAAAGT TGCAGGACCA CTTCTGCGCT CGGCCCTTCC GGCTGGCTGG TTTATTGCTG ATAAATCTGG AGCCGGTGAG
CGTGGGTCTC GCGGTATCAT TGCAGCACTG GGGCCAGATG GTAAGCCCTC CCGTATCGTA GTTATCTACA CGACGGGGAG
TCAGGCAACT ATGGATGAAC GAAATAGACA GATCGCTGAG ATAGGTGCCT CACTGATTAA GCATTGGTAA CTGTCAGACC
AAGTTTACTC ATATATACTT TAGATTGATT TAAAACTTCA TTTTTAATTT AAAAGGATCT AGGTGAAGAT CCTTTTTGAT
AATCTCATGA CCAAAATCCC TTAACGTGAG TTTTCGTTCC ACTGAGCGTC AGACCCCGTA GAAAAGATCA AAGGATCTTC
TTGAGATCCT TTTTTTCTGC GCGTAATCTG CTGCTTGCAA ACAAAAAAAC CACCGCTACC AGCGGTGGTT TGTTTGCCGG
ATCAAGAGCT ACCAACTCTT TTTCCGAAGG TAACTGGCTT CAGCAGAGCG CAGATACCAA ATACTGTCCT TCTAGTGTAG
CCGTAGTTAG GCCACCACTT CAAGAACTCT GTAGCACCGC CTACATACCT CGCTCTGCTA ATCCTGTTAC CAGTGGCTGC
TGCCAGTGGC GATAAGTCGT GTCTTACCGG GTTGGACTCA AGACGATAGT TACCGGATAA GGCGCAGCGG TCGGGCTGAA
CGGGGGGTTC GTGCACACAG CCCAGCTTGG AGCGAACGAC CTACACCGAA CTGAGATACC TACAGCGTGA GCATTGAGAA
AGCGCCACGC TTCCCGAAGG GAGAAAGGCG GACAGGTATC CGGTAAGCGG CAGGGTCGGA ACAGGAGAGC GCACGAGGGA
GCTTCCAGGG GGAAACGCCT GGTATCTTTA TAGTCCTGTC GGGTTTCGCC ACCTCTGACT TGAGCGTCGA TTTTTGTGAT
GCTCGTCAGG GGGGCGGAGC CTATGGAAAA ACGCCAGCAA CGCGGCCTTT TTACGGTTCC TGGCCTTTTG CTGGCCTTTT
GCTCACATGT TCTTTCCTGC GTTATCCCCT GATTCTGTGG ATAACCGTAT TACCGCCTTT GAGTGAGCTG ATACCGCTCG
CCGCAGCCGA ACGACCGAGC GCAGCGAGTC AGTGAGCGAG GAAGCGGAAG AGCGCCCAAT ACGCAAACCG CCTCTCCCCG
 
CGCGTTGGCC GATTCATTAA TGCAGCTGGC ACGACAGGTT TCCCGACTGG AAAGCGGGCA GTGAGCGCAA CGCAATTAAT
GTGAGTTAGC TCACTCATTA GGCACCCCAG GCTTTACACT TTATGCTTCC GGCTCGTATG TTGTGTGGAA TTGTGAGCGG
ATAACAATTT CACACAGGAA ACAGCTATGA CCATGATTAC GCCAAGCTTG CCAAATTCTA TTTCAAGGAG ACAGTCATAA
TGAAATACCT ATTGCCTACG GCAGCCGCTG GATTGTTATT ACTCGCAGCA AGCGGCGCGC ATGCCGACAT TCAGATGACC
CAGTCTCCAG CCTCCCTTTC TGCGTCTGTG GGAGAAACTG TCACCATCAC ATGTCGAGCA AGTGGGAATA TTCACATTTA
TTTAGCATGG TATCAGCAGA AACAGGGAAA ATCTCCTCAG CTCCTGGTCT ATTATACAAC AACCTTAGCA GATGGTGTGC
CATCAAGGTT CAGTGGCAGT GGATCAGGAA CACAATATTC TCTCAAGATC AACAGCCTGC AACCTGAAGA TTTTGGGAGT
TATTACTGTC AACATTTTTG GAGTACTCCT CGGACGTTCG GTGGAGGGAC CAAGCTGGAG CTGAAACGTT CCGGAGGGTC
GACCATAACT TCGTATAATG TATACTATAC GAAGTTATCC TCGAGCGGTA CCGAGGTGAA GCTGGTGGAG TCAGGACCTG
GCCTGGTGGC GCCCTCACAG AGCCTGTCCA TCACATGCAC CGTCTCAGGG TTCTCATTAA CCGGCTATGG TGTAAACTGG
GTTCGCCAGC CTCCAGGAAA GGGTCTGGAG TGGCTGGGAA TGATTTGGGG TGATGGAAAC ACAGACTATA ATTCAGCTCT
CAAATCCAGA CTGAGCATCA GCAAGGACAA CTCCAAGAGC CAAGTTTTCT TAAAAATGAA CAGTCTGCAC ACTGATGACA
CAGCCGTCTA CTACTGCGCG CGAGAGAGAG ATTATAGGCT TGACTACTGG GGCCAAGGCA CCACGGTCAC CGTCTCCTCA
G
Sequence ID No: 4. His6
   HHHHHH
Sequence ID No: 5. VHback-DAN
   TTA TCC TCG AGC GGT ACC SAG GTS MAR CTG CAG SAG TCW GG
Sequence ID No: 6. VHfor2-DAN
   GAT TGG TTT GCC GCT AGC TGA GGA GAC GGT GAC CGT GGT
Sequence ID No: 7. VK2back-DAN
   AGC AAG CGG CGC GCA TGC CGA CAT CGA GCT CAC CCA GTC TC 
Sequence ID No: 8. VK2for-DAN

GAA GTT ATG GTC GAC CCT CCG GAA CGT TTG ATC TCG AGC TTG
GTC CC
Sequence ID No: 9. VLbackPT1
   CGC TGG ATT GTT ATT ACT CGC AGC AAG CGG CGC GCA TGC C
Sequence ID No: 10. VLbackPT2
   TAC CTA TTG CCT ACG GCA GCC GCT GGA TTG TTA TTA CTC
Sequence ID No: 11. VHforPT1
   CCA GGC CCA GCA GTG GGT TTG GGA TTG GTT TGC CGC TA
Sequence ID No: 12, VHforPT2
   TGG TGA TGG TGA GTA CTA TCC AGG CCC AGC AGT GGG TTT G

Sequence ID No: 13.  VLforPTL

ACC GCT CGA GGA TAA CTT CGT ATA GTA TAC ATT ATA CGA AGT
TAT GGT CGA CCC TCC

Sequence ID No: 14.  VHbackPTL

GGA GGG TCG ACC ATA ACT TCG TAT AAT GTA TAC TAT ACG AAG
TTA TCC TCG AGC GGT A

## Claims

1. A method of preparing a nucleic acid library, said method comprising introducing at least two members of an initial population of nucleic acid molecules into at least one cell, said population of nucleic acid molecules comprising two or more individual nucleic acids each of which consists of a nucleic acid sequence that is the same for each molecule and that includes the same origin of replication; and a nucleic acid sequence that varies between members of said population and which comprises a substrate for recombination, said introducing resulting in recombination of said substrate for recombination between at least two members of the population thereby producing a population comprising recombined nucleic acid members.

2. The method of claim 1, wherein said recombination is performed by a recombination mechanism endogenous to said cell.

3. The method of claim 1, wherein said recombination is mediated by an exogenous recombinase.

4. The method of claim 1, wherein said recombination is mediated by an endogenous recombinase.

5. The method of claim 1, wherein said recombination is at a site preselected for recombination.

6. The method of claim 5, wherein the site preselected for recombination is a recombinase recognition site and the recombination is mediated by a recombinase expressed by said cell.

7. The method of claim 1, wherein said recombination is mediated by a recombinase selected from the group consisting of a member of the hin family of recombinases, a member of the lambda integrase family, an flp recombinase, a resolvase, a transposon, and a Cre recombinase.

8. The method of claim 7, wherein said recombinase is selected from the group consisting of Cre, hin, gin, pin, cin, and flp.

9. The method of claim 5, wherein said site preselected for recombination is a loxP site.

10. The method of claim 1, wherein said substrate for recombination, comprises a first site recombinase recognition site and a second recombinase recognition site different from the first recombinase recognition site.

11. The method of claim 10, wherein recombination results in the exchange, between two members of said nucleic acid population, of the nucleic acid flanked by the first and second recombinase recognition sites.

12. The method of claim 10, wherein the first recombinase recognition site is a loxP site and the second recombinase recognition site is a loxP mutant site.

13. The method of claim 12, wherein the loxP mutant site is loxP 511.

14. The method of claim 1, wherein said cell is selected from the group consisting of a bacterial cell, a yeast cell, an insect cell, and a mammalian cell.

15. The method of claim 14, wherein said bacterial cell is an *Escherichia coli* cell.

16. The method of claim 1, wherein said members of a population of nucleic acid molecules are introduced into the cell by transfection.

17. The method of claim 1, wherein said population of nucleic acid molecules comprises at least 10 different members.

18. The method of claims 1, wherein said members of a population of nucleic acid molecules are contained within infectious particles and are introduced into the cells via infection with said infectious particles.

19. The method of claim 18, wherein said infectious particles are phage.

20. The method of claim 19. wherein said infectious particles are filamentous phage.

21. The method of claim 20, wherein the infectious particles are filamentous phage of the Ff family.

22. The method of claim 18, wherein said infectious particles are phagemids containing phagemidic DNA.

23. The method of claim 18, wherein said infectious particles are phagemids derived from filamentous phage of the Ff family.

24. The method of claim 1, wherein said method further comprises :
transfecting or infecting one or more cells with members of said population of recombined nucleic acid members such that said cells are infected at a multiplicity of infection (moi) of less than about 1.

25. The method of claim 24, wherein said further method comprises the packaging the members of said nucleic acid library in replicable genetic display packages such that a protein on the surface of the replicable display package is encoded by a nucleic acid packaged within the display package that is a nucleic acid sequence that varies between members of the nucleic acid library.

26. The method of claims 1, wherein the variable nucleic acid sequence comprising the substrate for recombination comprises an expression cassette.

27. The method of claim 26, wherein said expression cassette comprises nucleic acid sequences encoding one or more polypeptides.

28. The method of claim 26, wherein said expression cassette comprises nucleic acid sequences encoding one or more polypeptides and the nucleic acid encoding at least one of said polypeptides is flanked by pair of recombinase recognition sites.

29. The method of claim 27, wherein said polypeptides are expressed on the surface of a phage, a phagemid, or a bacterium.

30. The method of claim 27, wherein said variable sequence includes nucleic acid encoding a first polypeptide chain and a second polypeptide chain from a specific binding pair member such that following recombination said variable sequence encodes binding proteins that are not present in the initial population of nucleic acids.

31. The method of claim 30, wherein said first and said second polypeptide are antibody polypeptides.

32. The method of claim 31, wherein said first and second polypeptide are selected from the group consisting of a V_{H} region, a V_{L} region, a V_{H} CDR1, a V_{H} CDR₂, a V_{H} CDR₃, a V_{L} CDR1, a V_{L} CDR₂, a V_{L} CDR₃, a VH joined to a C_{H}1, and a V_{L} joined to a C_{L}.

33. The method of claim 32, wherein the first polypeptide is a V_{H} region and the second polypeptide is a V_{L} region.

34. The method of claim 30, wherein a pair of recombinase recognition sites flank the nucleic acid encoding a first polypeptide and said pair of recombinase recognition sites comprise a first recombinase recognition site and a different second recombinase recognition site.

35. The method of claim 34, wherein the first recombinase recognition site is a LoxP site and the second recombinase recognition site is a LoxP 511 site.

36. The method of claim 30, wherein the first polypeptide is flanked by a pair of recombinase recognition sites and the recognition sites are different from each other.

37. The method of claim 30, wherein the first polypeptide and the second polypeptide are each flanked by a pair of recombinase recognition sites and the recognition sites within each pair are different from each other.

38. The method of claim 35, wherein said loxP sites are selected from the group consisting of loxP, loxP 511, and fas loxP.

39. The method of claim 38, wherein the members of said library encode a single-chain antibody.

40. The method of claim 38, in which said antibody fragments are scFv.

41. The method of claim 38, wherein the members of said library encode a moiety selected from the group consisting of a Fab, an Fv, a diabody, a V_{H} dimer, and a V_{L} dimer..

42. The method of claim 38, wherein the members of said library encode an antibody in which the antibody V regions are linked by a polypeptide linker comprising a recombinase recognition site.

43. The method of claim 42, wherein said recombinase recognition site is selected from the group consisting of loxP, a loxP mutant, a recognition site for a hin family recombinase, a recognition site for a lambda integrase, recognition site for an flp recombinase, a recognition site for a resolvase, and a recognition site for a transposon.

44. A method according to claim 1, wherein the variable portion of the nucleic acid further comprises a selectable marker whereby said selectable marker must be recombined with a second selectable marker to become active.

45. The method of claim 44 wherein said selectable marker is inactive without recombination because of mutations.

46. The method of claim 44 wherein said selectable marker is inactive without recombination because it is an incomplete selectable marker.

47. The method of claim 44 wherein said selectable marker is located such that it is linked to the recombination substrate and co-transferred with a gene of interest in said recombination substrate.

48. A nucleic acid library comprising a population of nucleic acid molecules comprising two or more individual nucleic acids each of which consists of a nucleic acid sequence that is the same for each molecule and that includes the same origin of replication and at least two recombinase recognition sites; and a nucleic acid sequence that varies between members of said population wherein said nucleic acid sequence that varies comprises a substrate for recombination, and wherein every member of said library has the same origin of replication.

49. The nucleic acid library of claim 48, wherein said library comprises at least 10 different members in a single cell.

50. The nucleic acid library of claim 49, wherein said library comprises at least 100 different members in a single cell.

51. The nucleic acid library of claim 48, wherein said recombinase recognition sites comprise sites recognized by a recombinase selected from the group consisting of a member of the hin family of recombinases, a member of the lambda integrase family, an flp recombinase, a resolvase, a transposon, and a Cre recombinase.

52. The nucleic acid library of claim 51, wherein said recombinase is selected from the group consisting of Cre, hin, gin, pin, cin, and flp.

53. The nucleic acid library of claim 52, wherein said recombinase recognition site is a LoxP site or a mutant LoxP site.

54. The nucleic acid library of claim 53, wherein said members of a population of nucleic acid molecules are contained within infectious particles.

55. The nucleic acid library of claim 54, wherein said infectious particles are phage.

56. The nucleic acid library of claim 55, wherein said infectious particles are filamentous phage.

57. The nucleic acid library of claim 56, wherein said infectious particles are filamentous phages of the Ff family.

58. The nucleic acid library of claim 54, wherein said infectious particles are phagemid containing phagemidic DNA.

59. The nucleic acid library of claim 58, wherein said infectious particles are phagemids derived from filamentous phage of the Ff family.

60. The nucleic acid library of claim 48, wherein the variable nucleic acid sequence comprising the substrate for recombination comprises an expression cassette.

61. The nucleic acid library of claim 60, wherein said expression cassette comprises nucleic acid sequences encoding one or more polypeptides.

62. The nucleic acid library of claim 61, wherein said polypeptides are expressed on the surface of a phage or phagemid.

63. The nucleic acid library of claim 48, wherein said variable sequence includes nucleic acid encoding a first polypeptide chain and a second polypeptide chain from a specific binding pair member such that following recombination binding nucleic acids encoding binding proteins are produced that are not present in the initial population of nucleic acids. encoding binding proteins are produced that are not present in the initial population of nucleic acids.

64. The nucleic acid library of claim 63, wherein said first and said second polypeptide are antibody polypeptides.

65. The nucleic acid library of claim 64, wherein said first and second polypeptide are selected from the group consisting of a V_{H} region, a V_{L} region, a.V_{H} CDR1, a V_{H} CDR₂, a V_{H} CDR₃, a V_{L} CDR1, a V_{L} CDR_{2,} a V_{L} CDR_{3,} a VH joined to a C_{H}1, and a V_{L} joined to a C_{L.}

66. The nucleic acid library of claim 65, wherein the first polypeptide is a V_{H} region and the second polypeptide is a V_{L} region.

67. The nucleic acid library of claim 63-66, wherein a pair of recombinase recognition sites flank the nucleic acid encoding a first polypeptide and pair of recombinase recognition sites comprise a first recombinase recognition site and a different second recombinase recognition site.

68. The nucleic acid library of claim 67, wherein the first recombinase recognition site is a LoxP site and the second recombinase recognition site is a LoxP 511 site.

69. The nucleic acid library of claim 48, wherein the members of said library encode a single-chain antibody.

70. The nucleic acid library of claims 63-66, wherein the first and the second polypeptide are expressed on the surface of a phage or bacterium.

71. The nucleic acid library of claim 70, in which said polypeptides are scFv, in which V_{H} and V_{L} are joined by a polypeptide linker encoded by a nucleic acid comprising a loxP, a loxP mutant, a recognition site for a hin family recombinase, a recognition site for a lambda integrase, recognition site for an flp recombinase, a recognition site for a resolvase, and a recognition site for a transposon.

72. The nucleic acid library of claims 48-66, wherein said nucleic acids express rgdp polypeptides.

73. A method of preparing a polypeptide said method comprising:
a) providing a nucleic acid library of claims 48-72;
b) selecting one or more members of said library; and
c) expressing the nucleic acids of the one or more selected members.

74. The method of claim 73, wherein said selecting comprises:
i) expressing proteins encoded by the members of said nucleic acid library; and
ii) screening the expressed proteins for one or more properties selected from the group consisting of specific binding to one or more preselected targets, a minimum binding avidity for one or more preselected targets, a maximum binding avidity for one or more preselected targets, thermostability at a particular preselected temperature, a predefined catalytic activity, a predefined enzymatic activity under selected conditions, a predefined biological activity; and
iii) selecting the library members that meet the screening criteria.

75. The method of claim 74, wherein said screening comprises screening for specific binding to a preselected target.

76. The method of claim 74, wherein the expressed proteins comprise single chain antibodies.

77. The method of claim 74, wherein the polypeptides are expressed on the surface of cells infected or transfected with the members of the nucleic acid library.

78. The method of claim 74, wherein the polypeptides are expressed on the surface of replicable genetic display packages (rgdp).

79. The method of claim 74 where said members selected in step (iii) are used to enrich or generate a library according to the method of claim 1.

80. The method of claim 78, in which said replicable genetic display package (rgdp) is a phagemid expressing one or more polypeptides bound to surface proteins.

81. A host cell comprising a nucleic acid library according to claims 48-72.

82. The host cell of claim 81, wherein said cell is selected from the group consisting of a bacterial cell, a plant cell, a mammalian cell, an insect cell, and a yeast cell.

## Patentansprüche

1. Verfahren für das Zubereiten einer Nukleinsäurebibliothek, wobei das Verfahren das Einführen von mindestens zwei Mitgliedern einer anfänglichen Population von Nukleinsäuremolekülen in mindestens eine Zelle umfasst, wobei die Population von Nukleinsäuremolekülen zwei oder mehr einzelne Nukleinsäuren umfasst, von denen jede aus einer Nukleinsäuresequenz besteht, die bei jedem Molekül gleich ist und die den gleichen Replikationsursprung einschließt; und eine Nukleinsäuresequenz, die zwischen Mitgliedern der Population variiert und ein Substrat für die Rekombination umfasst, wobei das Einführen zur Rekombination des Substrats zur Rekombination zwischen mindestens zwei Mitgliedern der Population führt, wodurch eine Population erzeugt wird, die rekombinierte Nukleinsäuremitglieder umfasst.

2. Verfahren nach Anspruch 1, wobei die Rekombination durch einen der Zelle endogenen Rekombinationsmechanismus durchgeführt wird.

3. Verfahren nach Anspruch 1, wobei die Rekombination durch eine exogene Rekombinase vermittelt wird.

4. Verfahren nach Anspruch 1, wobei die Rekombination durch eine endogene Rekombinase vermittelt wird.

5. Verfahren nach Anspruch 1, wobei die Rekombination an einer für die Rekombination vorgewählten Stelle stattfindet.

6. Verfahren nach Anspruch 5, wobei die für die Rekombination vorgewählte Stelle eine Rekombinaseerkennungsstelle ist und die Rekombination durch eine Rekombinase vermittelt wird, die durch die Zelle exprimiert wird.

7. Verfahren nach Anspruch 1, wobei die Rekombination durch eine Rekombinase vermittelt wird, die aus der Gruppe ausgewählt ist bestehend aus einem Mitglied der hin-Familie von Rekombinasen, einem Mitglied der Lambda-Integrasefamilie, einer flp-Rekombinase, einer Resolvase, einem Transposon und einer Cre-Rekombinase.

8. Verfahren nach Anspruch 7, wobei die Rekombinase aus der Gruppe ausgewählt wird bestehend aus Cre, hin, gin, pink, cin und flp.

9. Verfahren nach Anspruch 5, wobei die für die Rekombination ausgewählte Stelle eine loxP-Stelle ist.

10. Verfahren nach Anspruch 1, wobei das Substrat für die Rekombination eine erste Stellenrekombinaseerkennungsstelle und eine zweite Rekombinaseerkennungsstelle umfasst, die von der ersten Rekombinaseerkennungsstelle verschieden ist.

11. Verfahren nach Anspruch 10, wobei die Rekombinase zum Austausch zwischen zwei Mitgliedern der Nukleinsäurepopulation der durch die ersten und zweiten Rekombinaseerkennungsstellen flankierten Nukleinsäure führt.

12. Verfahren nach Anspruch 10, wobei die erste Rekombinaseerkennungsstelle eine loxP-Stelle und die zweite Rekombinaseerkennungsstelle eine mutante loxP-Stelle ist.

13. Verfahren nach Anspruch 12, wobei die mutante loxP-Stelle loxP 511 ist.

14. Verfahren nach Anspruch 1, wobei die Zelle aus der Gruppe ausgewählt ist bestehend aus einer Bakterienzelle, einer Hefezelle, einer Insektenzelle und einer Säugerzelle.

15. Verfahren nach Anspruch 14, wobei die Bakterienzelle eine Escherichia coli-Zelle ist.

16. Verfahren nach Anspruch 1, wobei die Mitglieder einer Population von Nukleinsäuremolekülen durch Transfizierung in die Zelle eingeführt werden.

17. Verfahren nach Anspruch 1, wobei die Population von Nukleinsäuremolekülen mindestens 10 verschiedene Mitglieder umfasst.

18. Verfahren nach Anspruch 1, wobei die Mitglieder einer Population von Nukleinsäuremolekülen innerhalb infektiöser Teilchen enthalten und in die Zellen durch Infektion mit den infektiösen Teilchen eingeführt werden.

19. Verfahren nach Anspruch 18, wobei die infektiösen Teilchen ein Phage sind.

20. Verfahren nach Anspruch 19, wobei die infektiösen Teilchen eines filamentösen Phagen sind.

21. Verfahren nach Anspruch 20, wobei die infektiösen Teilchen der filamentöse Phage der Ff-Familie sind.

22. Verfahren nach Anspruch 18, wobei die infektiösen Teilchen Phagemide sind, die phagemidische DNA enthalten.

23. Verfahren nach Anspruch 18, wobei die infektiösen Teilchen Phagemide sind, die von filamentösem Phage der Ff-Familie deriviert sind.

24. Verfahren nach Anspruch 1, wobei das Verfahren des Weiteren Folgendes umfasst:
Transfizieren oder Infizieren einer oder mehrerer Zellen mit Mitgliedern der Population von rekombinierten Nukleinsäuremitgliedern, derart, dass die Zellen bei einer Infektionsmultiplizität (moi) von weniger als ca. 1 infiziert sind.

25. Verfahren nach Anspruch 24, wobei das Verfahren des Weiteren das Packen der Mitglieder der Nukleinsäurebibliothek in replizierbare genetische Anzeigepakete umfasst, derart, dass ein Protein an der Oberfläche des replizierbaren Anzeigepakets durch eine Nukleinsäure kodiert wird, die innerhalb des Anzeigepakets gepackt ist, die eine Nukleinsäuresequenz ist, die zwischen Mitglieder der Nukleinsäurebibliothek variiert.

26. Verfahren nach Anspruch 1, wobei die variable Nukleinsäuresequenz, die das Substrat für die Rekombination umfasst, eine Expressionskassette umfasst.

27. Verfahren nach Anspruch 26, wobei die Expressionskassette Nukleinsäuresequenzen umfasst, die für ein oder mehrere Polypeptide kodieren.

28. Verfahren nach Anspruch 26, wobei die Expressionskassette Nukleinsäuresequenzen umfasst, die für ein oder mehrere Polypeptide kodieren und die Nukleinsäure, die für mindestens eines der Polypeptide kodiert, durch Paare von Rekombinaseerkennungsstellen flankiert sind.

29. Verfahren nach Anspruch 27, wobei die Polypeptide auf der Oberfläche eines Phagen, eines Phagemids oder einer Bakterie exprimiert sind.

30. Verfahren nach Anspruch 27, wobei die variable Sequenz Nukleinsäure umfasst, die für eine erste Polypeptidkette und eine zweite Polypeptidkette aus einem spezifischen Bindungspaarmitglied derart kodiert, dass auf die Rekombination hin die variable Sequenz für Bindungsproteine kodiert, die nicht in der ursprünglichen Population von Nukleinsäuren vorliegen.

31. Verfahren nach Anspruch 30, wobei die ersten und zweiten Polypeptide Antikörperpolypeptide sind.

32. Verfahren nach Anspruch 31, wobei das erste und zweite Polypeptid aus der Gruppe ausgewählt sind bestehend aus einer V_{H}-Region, einer V_{L}-Region, einem V_{H}CDR₁, einem V_{H}CDR₂, einem V_{H}CDR₃, einem V_{L}CDR₁, einem V_{L}CDR₂, einem V_{L}CDR₃, einem an ein CH₁ gebundenen V_{H} und einem an C_{L} gebundenen V_{L}.

33. Verfahren nach Anspruch 32, wobei das erste Polypeptid eine V_{H}-Region und das zweite Polypeptid eine V_{L}-Region ist.

34. Verfahren nach Anspruch 30, wobei ein Paar Rekombinaseerkennungsstellen die Nukleinsäure flankiert, die für ein erstes Polypeptid kodiert und das Paar Rekombinaseerkennungsstellen eine erste Rekombinaseerkennungsstelle und eine andere zweite Rekombinaseerkennungsstelle umfasst.

35. Verfahren nach Anspruch 34, wobei die erste Rekombinaseerkennungsstelle eine loxP-Stelle und die zweite Rekombinaseerkennungsstelle eine loxP 511-Stelle ist.

36. Verfahren nach Anspruch 30, wobei das erste Polypeptid von einem Paar Rekombinaseerkennungsstellen flankiert ist und die Erkennungsstellen voneinander verschieden sind.

37. Verfahren nach Anspruch 30, wobei das erste Polypeptid und das zweite Polypeptid jeweils von einem Paar Rekombinaseerkennungsstellen flankiert sind und die Erkennungsstellen innerhalb jedes Paars voneinander verschieden sind.

38. Verfahren nach Anspruch 35, wobei die loxP-Stellen aus der Gruppe ausgewählt sind bestehend aus loxP, loxP 511 und fas loxP.

39. Verfahren nach Anspruch 38, wobei die Mitglieder der Bibliothek für einen Einkettenantikörper kodieren.

40. Verfahren nach Anspruch 38, wobei die Antikörperfragmente scFv sind.

41. Verfahren nach Anspruch 38, wobei die Mitglieder der Bibliothek für einen Anteil kodieren, der aus der Gruppe ausgewählt ist bestehend aus einem Fab, einem Fv, einem Diakörper, einem V_{H}-Dimer und einem V_{L-}Dimer.

42. Verfahren nach Anspruch 38, wobei die Mitglieder der Bibliothek für einen Antikörper kodieren, bei dem die Antikörper-V-Regionen durch einen Polypeptidkoppler verkoppelt sind, der eine Rekombinaseerkennungsstelle umfasst.

43. Verfahren nach Anspruch 42, wobei die Rekombinaseerkennungsstelle aus der Gruppe ausgewählt ist bestehend aus loxP, einem loxP-Mutanten, einer Erkennungsstelle für eine hin-Familienrekombinase, einer Erkennungsstelle für eine Lambda-Integrase, einer Erkennungsstelle für eine flp-Rekombinase, einer Erkennungsstelle für eine Resolvase und einer Erkennungsstelle für ein Transposon.

44. Verfahren nach Anspruch 1, wobei der variable Anteil der Nukleinsäure des Weiteren einen auswählbaren Marker umfasst, wobei der auswählbare Marker mit einem zweiten auswählbaren Marker rekombiniert werden muss, um aktiv zu werden.

45. Verfahren nach Anspruch 44, wobei der auswählbare Marker ohne Rekombination aufgrund von Mutationen inaktiv ist.

46. Verfahren nach Anspruch 44, wobei der auswählbare Marker ohne Rekombination inaktiv ist, weil er ein unvollständiger auswählbarer Marker ist.

47. Verfahren nach Anspruch 44, wobei der auswählbare Marker derart positioniert ist, dass er mit dem Rekombinationssubstrat verknüpft ist und mit einem Gen, das in dem Rekombinationssubstrat von Interesse ist, gleichzeitig übertragen wird.

48. Nukleinsäurebibliothek umfassend eine Population von Nukleinsäuremolekülen umfassend zwei oder mehr einzelne Nukleinsäuren, von denen jede aus einer Nukleinsäuresequenz besteht, die bei jedem Molekül gleich ist und die den gleichen Replikationsursprung und mindestens zwei Rekombinaseerkennungsstellen einschließt; und eine Nukleinsäuresequenz, die zwischen Mitgliedern der Population variiert, wobei die Nukleinsäuresequenz, die variiert, ein Substrat für die Rekombination umfasst, und wobei jedes Mitglied der Bibliothek den gleichen Replikationsurspruch aufweist.

49. Nukleinsäurebibliothek nach Anspruch 48, wobei die Bibliothek mindestens 10 verschiedene Mitglieder in einer einzigen Zelle umfasst.

50. Nukleinsäurebibliothek nach Anspruch 48, wobei die Bibliothek mindestens 100 verschiedene Mitglieder in einer einzigen Zelle umfasst.

51. Nukleinsäurebibliothek nach Anspruch 48, wobei die Rekombinaseerkennungsstellen Stellen umfassen, die durch eine Rekombinase erkannt werden, die aus der Gruppe ausgewählt ist bestehend aus einem Mitglied der hin-Familie von Rekombinasen, einem Mitglied der Lambda-Integrasefamilie, einer flp-Rekombinase, einer Resolvase, einem Transposon und einer Cre-Rekombinase.

52. Nukleinsäurebibliothek nach Anspruch 51, wobei die Rekombinase aus der Gruppe ausgewählt ist bestehend aus Cre, hin, gin, pin, cin und flp.

53. Nukleinsäurebibliothek nach Anspruch 52, wobei die Rekombinaseerkennungsstelle eine loxP-Stelle oder eine mutante loxP-Stelle ist.

54. Nukleinsäurebibliothek nach Anspruch 53, wobei die Mitglieder einer Population von Nukleinsäuremolekülen innerhalb infektiöser Teilchen enthalten sind.

55. Nukleinsäurebibliothek nach Anspruch 54, wobei die infektiösen Teilchen Phage sind.

56. Nukleinsäurebibliothek nach Anspruch 55, wobei die infektiösen Teilchen filamentöser Phage sind.

57. Nukleinsäurebibliothek nach Anspruch 56, wobei die infektiösen Teilchen filamentöse Phagen der Ff-Familie sind.

58. Nukleinsäurebibliothek nach Anspruch 54, wobei die infektiösen Teilchen Phagemid sind, der phagemidische DNA enthält.

59. Nukleinsäurebibliothek nach Anspruch 58, wobei die infektiösen Teilchen Phagemide sind, die von filamentösem Phage der Ff-Familie deriviert sind.

60. Nukleinsäurebibliothek nach Anspruch 48, wobei die variable Nukleinsäuresequenz, die das Substrat für die Rekombination umfasst, eine Expressionskassette umfasst.

61. Nukleinsäurebibliothek nach Anspruch 60, wobei die Expressionskassette Nukleinsäuresequenzen umfasst, die für ein oder mehrere Polypeptide kodieren.

62. Nukleinsäurebibliothek nach Anspruch 61, wobei die Polypeptide auf der Oberfläche eines Phagen oder eines Phagemids exprimiert werden.

63. Nukleinsäurebibliothek nach Anspruch 48, wobei die variable Sequenz Nukleinsäure einschließt, die für eine erste Polypeptidkette und eine zweite Polypeptidkette aus einem spezifischen Bindungspaarmitlied derart kodiert, dass auf die Rekombination hin Bindungsnukleinsäuren, die für Bindungsproteine kodieren, erzeugt werden, die nicht in der ursprünglichen Population von Nukleinsäuren vorhanden sind.

64. Nukleinsäurebibliothek nach Anspruch 63, wobei das erste und das zweite Polypeptid Antikörperpolypeptide sind.

65. Nukleinsäurebibliothek nach Anspruch 64, wobei das erste und zweite Polypeptid aus der Gruppe ausgewählt sind bestehend aus einer V_{H}-Region, einer V_{L}-Region, einem V_{H}CDR₁, einem V_{H}CDR₂, einem V_{H}CDR₃, einem V_{L}CDR₁, einem V_{L}CDR₂, einem V_{L}CDR₃, einem an CH₁ gebundenen V_{H} und einem an C_{L} gebundenen V_{L}.

66. Nukleinsäurebibliothek nach Anspruch 65, wobei das erste Polypeptid eine V_{H}-Region und das zweite Polypeptid eine V_{L}-Region ist.

67. Nukleinsäurebibliothek nach Anspruch 63-66, wobei ein Paar Rekombinaseerkennungsstellen die Nukleinsäure flankiert, die für ein erstes Polypeptid kodiert und das Paar Rekombinaseerkennungsstellen eine erste Rekombinaseerkennungsstelle und eine andere zweite Rekombinaseerkennungsstelle umfasst.

68. Nukleinsäurebibliothek nach Anspruch 67, wobei die erste Rekombinaseerkennungsstelle eine LoxP-Stelle und die zweite Rekombinaseerkennungsstelle eine LoxP 511-Stelle ist.

69. Nukleinsäurebibliothek nach Anspruch 48, wobei die Mitglieder der Bibliothek für einen Einkettenantikörper kodieren.

70. Nukleinsäurebibliothek nach den Ansprüchen 63-66, wobei das erste und das zweite Polypeptid auf der Oberfläche eines Phagen oder einer Bakterie exprimiert sind.

71. Nukleinsäurebibliothek nach Anspruch 70, wobei die Polypeptide scFv sind, wobei VH und VL durch einen Polypeptidverknüpfer verbunden sind, der durch eine Nukleinsäure kodiert wird, die ein loxP, einen loxP-Mutanten, eine Erkennungsstelle für eine hin-Familienrekombinase, eine Erkennungsstelle für eine Lambda-Integrase, eine Erkennungsstelle für eine flp-Rekombinase, eine Erkennungsstelle für eine Resolvase und eine Erkennungsstelle für einen Transposon umfasst.

72. Nukleinsäurebibliothek nach Ansprüchen 48 - 66, wobei die Nukleinsäuren rgdp-Polypeptide exprimieren.

73. Verfahren für das Zubereiten eines Polypeptids, wobei das Verfahren folgendes umfasst:
a) Bereitstellen einer Nukleinsäurebibliothek nach den Ansprüchen 48 - 72;
b Auswählen eines oder mehrerer Mitglieder der Bibliothek;
c) Exprimieren der Nukleinsäuren des einen oder mehrerer ausgewählter Mitglieder.

74. Verfahren nach Anspruch 73, wobei das Auswählen Folgendes umfasst:
i) Exprimieren von Proteinen, die durch die Mitglieder der Nukleinsäurebibliothek kodiert sind; und
ii) Screenen der exprimierten Proteine bezüglich einer oder mehrerer Eigenschaften ausgewählt aus der Gruppe bestehend aus spezifischem Binden an ein oder mehrere vorgewählte Targets, einer Mindestbindungsavidität für ein oder mehrere vorgewählte Targets, einer maximalen Bindungsavidität für ein oder mehrere vorgewählte Targets, Thermostabilität bei einer spezifischen vorgewählten Temperatur, einer vordefinierten katalytischen Aktivität, einer vordefinierten enzymatischen Aktivität unter ausgewählten Bedingungen, einer vordefinierten biologischen Aktivität; und
iii) Auswählen der Bibliotheksmitglieder, die den Kriterien des Screenens entsprechen.

75. Verfahren nach Anspruch 74, wobei das Screenen das Screenen auf spezifisches Binden an ein vorgewähltes Target umfasst.

76. Verfahren nach Anspruch 74, wobei die exprimierten Proteine Einkettenantikörper umfassen.

77. Verfahren nach Anspruch 74, wobei die Polypeptide auf der Oberfläche von Zellen exprimiert werden, die mit den Mitgliedern der Nukleinsäurebibliothek infiziert oder transfiziert sind.

78. Verfahren nach Anspruch 74, wobei die Polypeptide auf der Oberfläche replizierbarer genetischer Anzeipakete (rgdp) exprimiert sind.

79. Verfahren nach Anspruch 74, wobei die in Schritt (iii) ausgewählten Mitglieder zum Anreichern oder Bilden einer Bibliothek dem Verfahren von Anspruch 1 entsprechend verwendet werden.

80. Verfahren nach Anspruch 78, wobei das replizierbare genetische Anzeigepaket (rgdp) ein Phagemid ist, der ein oder mehrere Polypeptide exprimiert, die an Oberflächenproteine gebunden sind.

81. Wirtszelle umfassend eine Nukleinsäurebibliothek nach Anspruch 48 - 72.

82. Wirtszelle nach Anspruch 81, wobei die Zelle aus der Gruppe ausgewählt ist bestehend aus einer Bakterienzelle, einer Pflanzenzelle, einer Säugerzelle, einer Insektenzelle und einer Hefezelle.

## Revendications

1. Procédé de préparation d'une banque d'acides nucléiques, ledit procédé comprenant l'introduction d'au moins deux membres d'une population initiale de molécules d'acides nucléiques dans au moins une cellule, ladite population de molécules d'acides nucléiques comprenant deux ou davantage d'acides nucléiques individuels dont chacun est constitué d'une séquence d'acide nucléique qui est identique pour chaque molécule et qui comprend la même origine de réplication ; et d'une séquence d'acide nucléique qui varie entre les membres de ladite population et qui comprend un substrat de recombinaison, ladite introduction entraînant la recombinaison dudit substrat de recombinaison entre au moins deux membres de la population en produisant ainsi une population comprenant des membres d'acides nucléiques recombinés.

2. Procédé selon la revendication 1, dans lequel ladite recombinaison est effectuée par le biais d'un mécanisme de recombinaison endogène à ladite cellule.

3. Procédé selon la revendication 1, dans lequel ladite recombinaison est médiée par une recombinase exogène.

4. Procédé selon la revendication 1, dans lequel ladite recombinaison est médiée par une recombinase endogène.

5. Procédé selon la revendication 1, dans lequel ladite recombinaison est située au niveau du site présélectionné de recombinaison.

6. Procédé selon la revendication 5, dans lequel le site présélectionné de recombinaison est un site de reconnaissance de la recombinase et la recombinaison est médiée par une recombinase exprimée par ladite cellule.

7. Procédé selon la revendication 1, dans lequel ladite recombinaison est médiée par une recombinase sélectionnée dans le groupe constitué d'un membre de la famille de recombinases hin, d'un membre de la famille des intégrases lambda, d'une recombinase flp, d'une résolvase, d'un transposon et d'une recombinase Cre.

8. Procédé selon la revendication 7, dans lequel ladite recombinase est sélectionnée dans le groupe constitué de Cre, hin, gin, pin, cin et flp.

9. Procédé selon la revendication 5, dans lequel ledit site présélectionné de recombinaison est un site loxP .

10. Procédé selon la revendication 1, dans lequel ledit substrat de recombinaison comprend un premier site de reconnaissance de la recombinase et un deuxième site de reconnaissance de la recombinase différent du premier site de reconnaissance de la recombinase.

11. Procédé selon la revendication 10, dans lequel la recombinaison entraîne l'échange, entre deux membres de ladite population d'acides nucléiques, de l'acide nucléique flanqué par les premier et deuxième sites de reconnaissance de la recombinase.

12. Procédé selon la revendication 10, dans lequel le premier site de reconnaissance de la recombinase est un site loxP et le deuxième site de reconnaissance de la recombinase est un site mutant loxP.

13. Procédé selon la revendication 12, dans lequel le site mutant loxP est loxP 511.

14. Procédé selon la revendication 1, dans lequel ladite cellule est sélectionnée dans le groupe constitué d'une cellule bactérienne, d'une cellule de levure, d'une cellule d'insecte et d'une cellule de mammifère.

15. Procédé selon la revendication 14, dans lequel ladite cellule bactérienne est une cellule *d'Escherichia coli.*

16. Procédé selon la revendication 1, dans lequel lesdits membres d'une population de molécules d'acides nucléiques sont introduits dans la cellule par transfection.

17. Procédé selon la revendication 1, dans lequel ladite population de molécules d'acides nucléiques comprend au moins 10 membres différents.

18. Procédé selon la revendication 1, dans lequel lesdits membres d'une population de molécules d'acides nucléiques sont contenus dans des particules infectieuses et sont introduits dans les cellules par infection avec lesdites particules infectieuses.

19. Procédé selon la revendication 18, dans lequel lesdites particules infectieuses sont un phage.

20. Procédé selon la revendication 19, dans lequel lesdites particules infectieuses sont un phage filamenteux.

21. Procédé selon la revendication 20, dans lequel lesdites particules infectieuses sont un phage filamenteux de la famille Ff.

22. Procédé selon la revendication 18, dans lequel lesdites particules infectieuses sont des phagémides contenant de l'ADN phagémidique.

23. Procédé selon la revendication 18, dans lequel lesdites particules infectieuses sont des phagémides dérivés du phage filamenteux de la famille Ff.

24. Procédé selon la revendication 1, dans lequel ledit procédé comprend en outre :
la transfection ou l'infection d'une ou plusieurs cellules avec des membres de ladite population de membres d'acides nucléiques recombinés de sorte que lesdites cellules sont infectées à une multiplicité d'infection (moi) de moins d'environ 1.

25. Procédé selon la revendication 24, dans lequel ledit procédé comprend en outre l'encapsidation des membres de ladite banque d'acides nucléiques dans des capsides de présentation génétiques réplicables de sorte qu'une protéine sur la surface de la capside de présentation réplicable est codée par un acide nucléique encapsidé dans la capside de présentation qui est une séquence d'acide nucléique qui varie entre les membres de la banque d'acides nucléiques.

26. Procédé selon la revendication 1, dans lequel la séquence d'acide nucléique variable comprenant le substrat de recombinaison comprend une cassette d'expression.

27. Procédé selon la revendication 26, dans lequel ladite cassette d'expression comprend des séquences d'acides nucléiques codant un ou plusieurs polypeptides.

28. Procédé selon la revendication 26, dans lequel ladite cassette d'expression comprend des séquences d'acides nucléiques codant un ou plusieurs polypeptides et l'acide nucléique codant au moins un desdits polypeptides est flanqué par une paire de sites de reconnaissance de la recombinase.

29. Procédé selon la revendication 27, dans lequel lesdits polypeptides sont exprimés sur la surface d'un phage, d'un phagémide ou d'une bactérie.

30. Procédé selon la revendication 27, dans lequel ladite séquence variable comprend un acide nucléique codant une première chaîne polypeptidique et une deuxième chaîne polypeptidique issues d'un membre spécifique de la paire de liaison de sorte qu'après la recombinaison, ladite séquence variable code les protéines de liaison qui ne sont pas présentes dans la population initiale d'acides nucléiques.

31. Procédé selon la revendication 30, dans lequel lesdits premier et deuxième polypeptides sont des polypeptides d'anticorps.

32. Procédé selon la revendication 31, dans lequel lesdits premier et deuxième polypeptides sont sélectionnés dans le groupe constitué d'une région V_{H}, d'une région V_{L}, d'une CDR1 de V_{H}, d'une CDR₂ de V_{H}, d'une CDR₃ de V_{H}, d'une CDR1 de V_{L}, d'une CDR₂ de V_{L}, d'une CDR₃ de V_{L}, d'une V_{H} liée à un C_{H}1 et d'une V_{L} liée à un C_{L}.

33. Procédé selon la revendication 32, dans lequel le premier polypeptide est une région V_{H} et le deuxième polypeptide est une région V_{L}.

34. Procédé selon la revendication 30, dans lequel une paire de sites de reconnaissance de la recombinase flanque l'acide nucléique codant un premier polypeptide et ladite paire de sites de reconnaissance de la recombinase comprend un premier site de reconnaissance de la recombinase et un deuxième site distinct de reconnaissance de la recombinase.

35. Procédé selon la revendication 34, dans lequel le premier site de reconnaissance de la recombinase est un site loxP et le deuxième site de reconnaissance de la recombinase est un site loxP 511.

36. Procédé selon la revendication 30, dans lequel le premier polypeptide est flanqué par une paire de sites de reconnaissance de la recombinase et les sites de reconnaissance sont différents l'un de l'autre.

37. Procédé selon la revendication 30, dans lequel le premier polypeptide et le deuxième polypeptide sont chacun flanqués par une paire de sites de reconnaissance de la recombinase et les sites de reconnaissance dans chaque paire sont différents l'un de l'autre.

38. Procédé selon la revendication 35, dans lequel lesdits sites loxP sont sélectionnés dans le groupe constitué de loxP, loxP 511 et fas loxP.

39. Procédé selon la revendication 38, dans lequel les membres de ladite banque codent un anticorps monocaténaire.

40. Procédé selon la revendication 38, dans lequel lesdits fragments d'anticorps sont scFv.

41. Procédé selon la revendication 38, dans lequel les membres de ladite banque codent un groupement sélectionné dans le groupe constitué d'un Fab, d'un Fv, d'une forme dimère, d'un dimère de V_{H} et d'un dimère de V_{L}.

42. Procédé selon la revendication 38, dans lequel les membres de ladite banque codent un anticorps dans lequel les régions V de l'anticorps sont liées par un lieur polypeptide comprenant un site de reconnaissance de la recombinase.

43. Procédé selon la revendication 42, dans lequel ledit site de reconnaissance de la recombinase est sélectionné dans le groupe constitué de loxP, d'un mutant loxP, d'un site de reconnaissance d'une recombinase de la famille hin, d'un site de reconnaissance d'une intégrase lambda, d'un site de reconnaissance d'une recombinase flp, d'un site de reconnaissance d'une résolvase et d'un site de reconnaissance d'un transposon.

44. Procédé selon la revendication 1, dans lequel la partie variable de l'acide nucléique comprend en outre un marqueur sélectionnable moyennant quoi ledit marqueur sélectionnable doit être recombiné avec un deuxième marqueur sélectionnable pour devenir actif.

45. Procédé selon la revendication 44, dans lequel ledit marqueur sélectionnable est inactif sans recombinaison en raison de mutations.

46. Procédé selon la revendication 44, dans lequel ledit marqueur sélectionnable est inactif sans recombinaison dans la mesure où il s'agit d'un marqueur sélectionnable incomplet.

47. Procédé selon la revendication 44, dans lequel ledit marqueur sélectionnable est situé de façon à être lié au substrat de recombinaison et cotransféré avec un gène d'intérêt dans ledit substrat de recombinaison.

48. Banque d'acides nucléiques comprenant une population de molécules d'acides nucléiques comprenant deux ou davantage d'acides nucléiques individuels dont chacun est constitué d'une séquence d'acide nucléique qui est identique pour chaque molécule et qui comprend la même origine de réplication et d'au moins deux sites de reconnaissance de la recombinase ; et une séquence d'acide nucléique qui varie entre les membres de ladite population dans laquelle ladite séquence d'acide nucléique qui varie comprend un substrat de recombinaison, et dans laquelle chaque membre de ladite banque a la même origine de réplication.

49. Banque d'acides nucléiques selon la revendication 48, dans laquelle ladite banque comprend au moins 10 membres différents dans une seule cellule.

50. Banque d'acides nucléiques selon la revendication 49, dans laquelle ladite banque comprend au moins 100 membres différents dans une seule cellule.

51. Banque d'acides nucléiques selon la revendication 48, dans laquelle lesdits sites de reconnaissance de la recombinase comprennent des sites reconnus par une recombinase sélectionnée dans le groupe constitué d'un membre de la famille des recombinases hin, d'un membre de la famille des intégrases lambda, d'une recombinase flp, d'une résolvase, d'un transposon et d'une recombinase Cre.

52. Banque d'acides nucléiques selon la revendication 51, dans laquelle ladite recombinase est sélectionnée dans le groupe constitué de Cre, hin, gin, pin, cin et flp.

53. Banque d'acides nucléiques selon la revendication 52, dans laquelle ledit site de reconnaissance de la recombinase est un site loxP ou un site mutant loxP.

54. Banque d'acides nucléiques selon la revendication 53, dans laquelle lesdits membres d'une population de molécules d'acides nucléiques sont contenus dans des particules infectieuses.

55. Banque d'acides nucléiques selon la revendication 54, dans laquelle lesdites particules infectieuses sont un phage.

56. Banque d'acides nucléiques selon la revendication 55, dans laquelle lesdites particules infectieuses sont un phage filamenteux.

57. Banque d'acides nucléiques selon la revendication 56, dans laquelle lesdites particules infectieuses sont des phages filamenteux de la famille Ff.

58. Banque d'acides nucléiques selon la revendication 54, dans laquelle lesdites particules infectieuses sont un phagémide contenant de l'ADN phagémidique.

59. Banque d'acides nucléiques selon la revendication 58, dans laquelle lesdites particules infectieuses sont des phagémides dérivés du phage filamenteux de la famille Ff.

60. Banque d'acides nucléiques selon la revendication 48, dans laquelle la séquence d'acide nucléique variable comprenant le substrat de recombinaison comprend une cassette d'expression.

61. Banque d'acides nucléiques selon la revendication 60, dans laquelle ladite cassette d'expression comprend des séquences d'acides nucléiques codant un ou plusieurs polypeptides.

62. Banque d'acides nucléiques selon la revendication 61, dans laquelle lesdits polypeptides sont exprimés sur la surface d'un phage ou d'un phagémide.

63. Banque d'acides nucléiques selon la revendication 48, dans laquelle ladite séquence variable comprend un acide nucléique codant une première chaîne polypeptidique et une deuxième chaîne polypeptidique issues d'un membre spécifique de la paire de liaison de sorte qu'après la recombinaison, des acides nucléiques de liaison codant les protéines de liaison sont produits, lesquels ne sont pas présents dans la population initiale d'acides nucléiques.

64. Banque d'acides nucléiques selon la revendication 63, dans laquelle lesdits premier et deuxième polypeptides sont des polypeptides d'anticorps.

65. Banque d'acides nucléiques selon la revendication 64, dans laquelle lesdits premier et deuxième polypeptides sont sélectionnés dans le groupe constitué d'une région V_{H}, d'une région V_{L}, d'une CDR1 de V_{H}, d'une CDR₂ de V_{H}, d'une CDR₃ de V_{H}, d'une CDR1 de V_{L}, d'une CDR₂ de V_{L}, d'une CDR₃ de V_{L}, d'une V_{H} liée à un C_{H}1 et d'une V_{L} liée à un C_{L}.

66. Banque d'acides nucléiques selon la revendication 65, dans laquelle le premier polypeptide est une région V_{H} et le deuxième polypeptide est une région V_{L}.

67. Banque d'acides nucléiques selon les revendications 63 à 66, dans laquelle une paire de sites de reconnaissance de la recombinase flanque l'acide nucléique codant un premier polypeptide et une paire de sites de reconnaissance de la recombinase comprend un premier site de reconnaissance de la recombinase et un deuxième site distinct de reconnaissance de la recombinase.

68. Banque d'acides nucléiques selon la revendication 67, dans laquelle le premier site de reconnaissance de la recombinase est un site LoxP et le deuxième site de reconnaissance de la recombinase est un site LoxP 511.

69. Banque d'acides nucléiques selon la revendication 48, dans laquelle les membres de ladite banque codent un anticorps monocaténaire.

70. Banque d'acides nucléiques selon les revendications 63 à 66, dans laquelle les premier et deuxième polypeptides sont exprimés sur la surface d'un phage ou d'une bactérie.

71. Banque d'acides nucléiques selon la revendication 70, dans laquelle lesdits polypeptides sont scFv, où V_{H} et V_{L} sont liées par un lieur polypeptide codé par un acide nucléique comprenant un site loxP, un site mutant loxP, un site de reconnaissance d'une recombinase de la famille hin, un site de reconnaissance d'une intégrase lambda, un site de reconnaissance d'une recombinase f1p, un site de reconnaissance d'une résolvase et un site de reconnaissance d'un transposon.

72. Banque d'acides nucléiques selon les revendications 48 à 66, dans laquelle lesdits acides nucléiques expriment des polypeptides rgdp.

73. Procédé de préparation d'un polypeptide, ledit procédé comprenant :
a) la fourniture d'une banque d'acides nucléiques selon les revendications 48 à 72 ;
b) la sélection d'un ou plusieurs membres de ladite banque ; et
c) l'expression des acides nucléiques de l'un ou des différents membres sélectionnés.

74. Procédé selon la revendication 73, dans lequel ladite sélection comprend :
i) l'expression des protéines codées par les membres de ladite banque d'acides nucléiques ; et
ii) le criblage des protéines exprimées pour une ou plusieurs propriétés sélectionnées dans le groupe constitué de la liaison spécifique à une ou plusieurs cibles présélectionnées, d'une avidité de liaison minimale pour une ou plusieurs cibles présélectionnées, d'une avidité de liaison maximale pour une ou plusieurs cibles présélectionnées, d'une thermostabilité à une température présélectionnée particulière, d'une activité catalytique prédéfinie, d'une activité enzymatique prédéfinie dans des conditions sélectionnées, d'une activité biologique prédéfinie ; et
iii) la sélection des membres de la banque qui répondent aux critères de criblage.

75. Procédé selon la revendication 74, dans lequel ledit criblage comprend le criblage pour une liaison spécifique à une cible présélectionnée.

76. Procédé selon la revendication 74, dans lequel les protéines exprimées comprennent des anticorps monocaténaires.

77. Procédé selon la revendication 74, dans lequel les polypeptides sont exprimés sur la surface de cellules infectées ou transfectées avec les membres de la banque d'acides nucléiques.

78. Procédé selon la revendication 74, dans lequel les polypeptides sont exprimés sur la surface de capsides de présentation génétiques réplicables (rgdp).

79. Procédé selon la revendication 74, dans lequel lesdits membres sélectionnés dans l'étape (iii) sont utilisés pour enrichir ou générer une banque conformément au procédé selon la revendication 1.

80. Procédé selon la revendication 78, dans lequel ladite capside de présentation génétique réplicable (rgdp) est un phagémide exprimant un ou plusieurs polypeptides liés à des protéines de surface.

81. Cellule hôte comprenant une banque d'acides nucléiques selon les revendications 48 à 72.

82. Cellule hôte selon la revendication 81, dans laquelle ladite cellule est sélectionnée dans le groupe constitué d'une cellule bactérienne, d'une cellule de plante, d'une cellule de mammifère, d'une cellule d'insecte et d'une cellule de levure.
